# EUROPEAN PATENT APPLICATION

(11) **EP 4 600 356 A1**
(43) Date of publication of application: **13.08.2025**
(21) Application number: 23874893.3
(22) Date of filing: 04.10.2023
(51) Int. Cl.: C12N 15/11, A61K 31/7088, A61K 47/42, A61K 48/00, A61P 43/00, C07K 7/06, C07K 7/08, C07K 14/00, C07K 14/435, C12N 15/12, C12N 15/87

(54) **METHOD FOR INTRODUCING NUCLEIC ACID INTO MITOCHONDRION**

(30) Priority: 04.10.2022 JP 2022160059
(71) Applicant: RIKEN, Wako-shi, Saitama 351-0198 (JP)
(72) Inventor: NUMATA Keiji, Wako-shi, Saitama 351-0198 (JP); YOSHINAGA Naoto, Wako-shi, Saitama 351-0198 (JP)
(74) Representative: Mewburn Ellis LLP
(86) International application number: PCT/JP2023/036182
(87) International publication number: WO 2024/075767

(57) **Abstract**

The purposes of the present invention are to provide a method of easily introducing a gene to mitochondria from the outside of a cell, and to provide a method of introducing a relatively large nucleic acid to mitochondria. A carrier peptide for introducing a nucleic acid to mitochondria is provided, which comprises a mitochondrial-targeting sequence and a polycationic sequence, wherein said mitochondrial-targeting sequence consists of an N-terminal region comprising a transmembrane domain in a mitoNEET protein.

## Description

### Technical Field

The present invention relates to a carrier peptide for introducing a nucleic acid to mitochondria, a carrier peptide / nucleic acid complex, a pharmaceutical composition for treating a mitochondrial disease, a method for producing a carrier peptide / nucleic acid complex, and a method of introducing a nucleic acid to mitochondria.

### Background Art

Mitochondria play important roles in intracellular energy production, control of reactive oxygen species, and the like.

Mitochondrial DNA (mtDNA), the genome of mitochondria themselves, encodes 13 kinds of proteins, which are important enzymes that function in oxidative phosphorylation in mitochondria. However, mtDNA is exposed to the oxidative environment in mitochondria and is not protected by histone proteins. Therefore, mtDNA is prone to undergo mutation, and as mutations accumulate with age, increased mitochondrial genetic variation may emerge within the same cell. This condition is called heteroplasmy, and is thought to cause a mitochondrial disease due to impaired normal expression of mitochondrial proteins encoded by mtDNA.

Methods for efficiently introducing a gene to mitochondria in order to treat a mitochondrial disease have been studied for many years. A known classical mitochondrial delivery method uses lipophilic cations such as triphenylphosphine as a mitochondrial-targeting module. This method uses electrostatic interactions with highly negatively charged mitochondrial membranes, but is known to have a limited effect on mitochondria and is associated with cytotoxicity because lipophilic cations can also promote interactions with other negatively charged biomacromolecules.

In recent years, mitochondrial gene therapy has been dominated by a technique of introducing a gene encoding a fusion protein, in which a protein of interest for treatment is fused with a mitochondrial-targeting sequence (MTS), to a nucleus, and transferring the expressed fusion protein to the mitochondria. This technique is called allotopic delivery, and clinical trials are underway for mitochondrial diseases. However, this technique is not a method of introducing a gene directly to mitochondria and is not sufficiently effective.

As the most efficient method among conventional techniques, a method that uses an adeno-associated virus (AAV) is known. This method involves substituting the capsid protein of AAV with MTS to facilitate the transfer of AAV to the mitochondria. It has been revealed that the use of this method enables the introduction of the NADH subunit 4 gene to mitochondria, followed by sufficient expression in the mitochondria. However, there is a limit to the size of DNA that can be carried by AAV, with approximately 5 kbp being the upper limit. In gene therapy for mitochondrial diseases, it is desirable to introduce multiple genes to control complicated mitochondrial functions, and especially to improve the heteroplasmy; it is ideal to introduce the entire 16.6kbp mtDNA. However, when AAV is used, it is not easy to manipulate AAV to carry multiple genes, including the entire mtDNA. Furthermore, the method using AAV is problematic in its complicated process of preparing AAV into which MTS has been introduced and cannot be easily carried out.

Therefore, there is a need for new technologies to easily perform gene transfer to mitochondria.

### Citation List

### Patent Literature

Patent Literature 1: WO2013/129698

### Non-patent Literature

Non-patent Literature 1: Chuah, J. A. et al., Biomacromolecules, 2016, 17(11):3547-3557.

### Summary of Invention

### Technical Problem

An object of the present invention is to provide a method of easily introducing a gene to mitochondria and a method of introducing a relatively large nucleic acid to mitochondria.

### Solution to Problem

In order to solve the above problems, the present inventors have prepared a carrier peptide / nucleic acid complex by: constructing a new carrier peptide as a fusion peptide prepared by combining a mitochondrial-targeting sequence comprising the transmembrane domain of a mitoNEET protein with a polycationic sequence; and then mixing the carrier peptide with a nucleic acid. The present inventors have discovered that through introduction of this carrier peptide / nucleic acid complex to a cell, the carrier peptide / nucleic acid complex can be efficiently transferred to mitochondria and the protein encoded by the nucleic acid can be expressed in the mitochondria.

Furthermore, the present inventors have prepared a carrier peptide/mitochondrial DNA (mtDNA) complex by mixing the above carrier peptide with mtDNA, and then introduced this to cells with mtDNA-depletion, so that they have succeeded in restoring mitochondrial function. The present invention provides the following based on the above findings.

(1) A carrier peptide for introducing a nucleic acid to mitochondria, comprising a mitochondrial-targeting sequence and a polycationic sequence,
   wherein said mitochondrial-targeting sequence consists of an N-terminal region comprising the transmembrane domain in a mitoNEET protein.
(2) The carrier peptide of (1), wherein said mitochondrial-targeting sequence consists of the amino acid sequence MSLTSSSSVRVEWIAAVTIAAGTAAIGYLAYK (SEQ ID NO: 2).
(3) The carrier peptide of (1) or (2), wherein said polycationic sequence comprises at least three cationic amino acid residues.
(4) The carrier peptide of (3), wherein said cationic amino acid residues are lysine (K) residues, arginine (R) residues, histidine (H) residues, and/or derivatives of any of them.
(5) The carrier peptide of (1) or (2), wherein said polycationic sequence comprises:
   five or more consecutive lysine (K) residues, arginine (R) residues, histidine (H) residues, or derivatives of any of them;
   seven or more consecutive lysine (K) residues, arginine (R) residues, histidine (H) residues, and/or derivatives of any of them; or
   a sequence formed by repeating the amino acid sequence KH or RH three to twenty times.
(6) A nucleic acid encoding the carrier peptide of any one of (1) to (5).
(7) A carrier peptide / nucleic acid complex for introducing a nucleic acid to mitochondria, comprising the carrier peptide of any one of (1) to (5) and the nucleic acid.
(8) The carrier peptide / nucleic acid complex of (7), wherein the number of amino groups and guanidino groups derived from said carrier peptide/the number of phosphate groups derived from said nucleic acid (N/P ratio) is 0.2 or more and 100 or less.
(9) The carrier peptide / nucleic acid complex of (7) or (8), wherein said nucleic acid encodes a mitochondrial protein.
(10) The carrier peptide / nucleic acid complex of any one of (7) to (9), wherein said nucleic acid is DNA or RNA.
(11) The carrier peptide / nucleic acid complex of (10), wherein said DNA is 1 kbp to 20 kbp.
(12) The carrier peptide / nucleic acid complex of (10) or (11), wherein said DNA is a mitochondrial DNA.
(13) A pharmaceutical composition for treating a mitochondrial disease, comprising the carrier peptide / nucleic acid complex of any one of (7) to (12).
(14) A method of producing a carrier peptide / nucleic acid complex, comprising:
   a complex formation step of mixing the carrier peptide of any one of (1) to (5) with a nucleic acid to form a carrier peptide / nucleic acid complex comprising said carrier peptide and said nucleic acid.
(15) A method of introducing a nucleic acid to mitochondria of an isolated target cell, comprising:
   a contact step of contacting the carrier peptide / nucleic acid complex of any one of (7) to (12) with an isolated target cell, and
   a transfer step of culturing said target cell after said contact step to transfer said carrier peptide / nucleic acid complex to mitochondria.

The present specification encompasses the disclosure of Japanese Patent Application No. 2022-160059, on which the priority of the present application is based.

### Advantageous Effects of Invention

The present invention provides a method of easily introducing a gene to mitochondria. Moreover, a relatively large nucleic acid can be introduced to mitochondria based on the present invention.

### Brief Description of Drawing

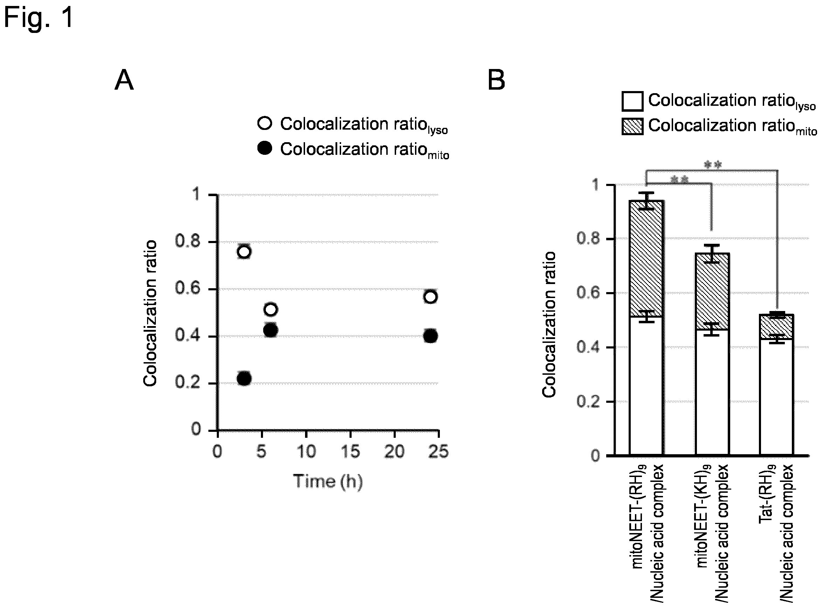

[Figure 1] Figure 1 depicts the colocalization ratio of a mitoNEET-(RH)₉/nucleic acid complex with mitochondria and the same with late endosomes/lysosomes. Figure 1A depicts the colocalization ratioₘᵢₜₒ of the mitoNEET-(RH)₉/nucleic acid complex with mitochondria and the colocalization ratio_{lyso} of the same with late endosomes/lysosomes at 3, 6, and 24 hours after transfection. Error bars indicate standard error (SEM). Figure 1B depicts the colocalization ratioₘᵢₜₒ and the colocalization ratio_{lyso} at 6 hours after transfection. Error bars indicate standard error (SEM). "**" indicates p<0.01 (one way ANOVA with Tukey's post-hoc test).

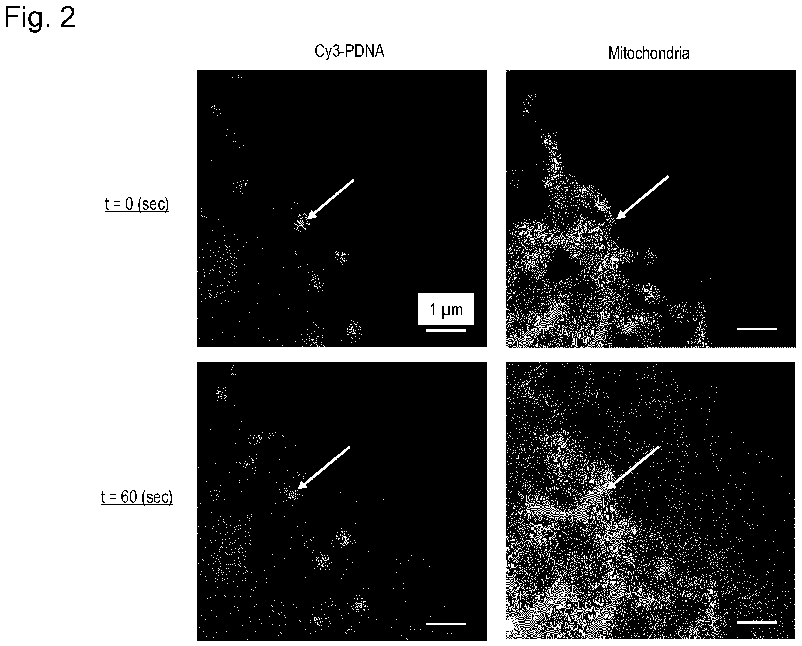

[Figure 2] Figure 2 depicts the time-lapse images of the transfer process of the mitoNEET-(RH)₉/nucleic acid complex to mitochondria. Arrows indicate the position of the mitoNEET-(RH)₉/nucleic acid complex. Scale bar indicates 1 µm.

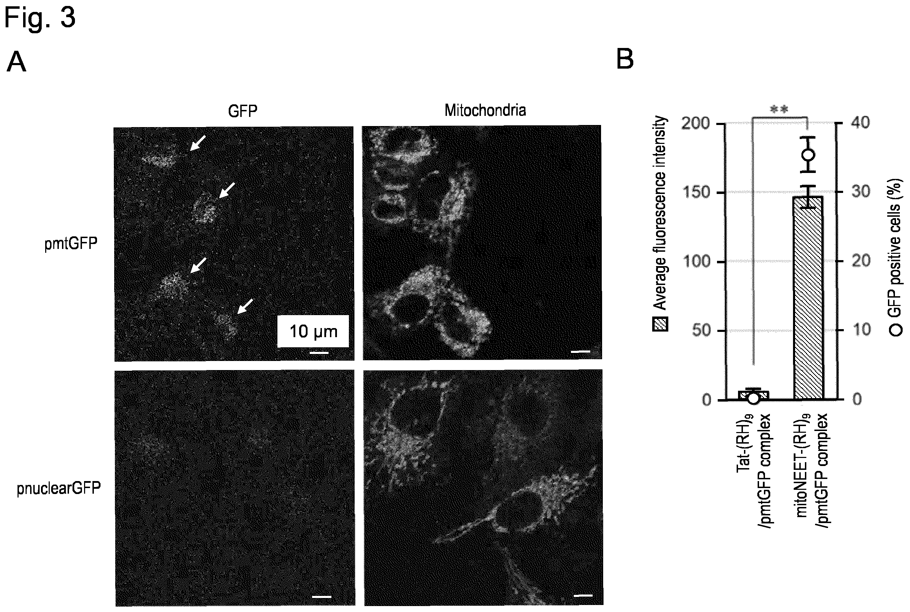

[Figure 3] Figure 3 depicts the expression of GFP (green fluorescent protein) in the mitochondria of Hela cells transfected with carrier peptide / nucleic acid complexes. Figure 3A depicts the expression of GFP (arrow) in the mitochondria of HeLa cells transfected with a mitoNEET-(RH)₉/pmtGFP complex and a mitoNEET-(RH)₉/pnuclearGFP complex (control). Scale bar indicates 10 µm. Figure 3B depicts the average fluorescence intensity based on GFP and the percentage of GFP positive cells in Hela cells transfected with the mitoNEET-(RH)₉/pmtGFP complex and a Tat-(RH)₉/pmtGFP complex (control). Error bars indicate standard error (SEM). "**" indicates p<0.01 (one way ANOVA with Dunnett's post-hoc test).

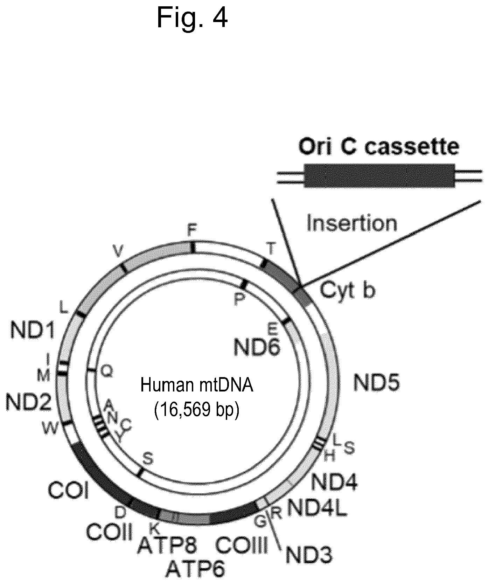

[Figure 4] Figure 4 depicts human mtDNA with an OriC cassette inserted for *in vitro* amplification.

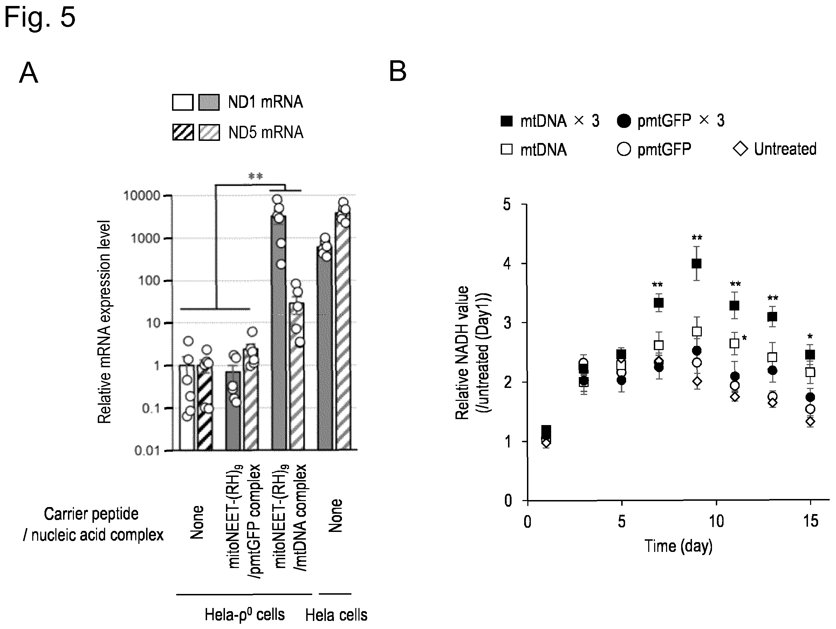

[Figure 5] Figure 5 depicts mitochondrial gene expression levels and the results of quantifying NADH levels in Hela-ρ⁰ cells transfected with carrier peptide / nucleic acid complexes. Figure 5A depicts the expression levels of ND1 mRNA and ND5 mRNA in untreated Hela-ρ⁰ cells, Hela-ρ⁰ cells transfected with the mitoNEET-(RH)₉/pmtGFP complex or a mitoNEET-(RH)₉/mtDNA complex, and Hela cells without mitochondrial defects. Error bars indicate standard error (SEM). "**" indicates p<0.01 (one way ANOVA with Tukey's post-hoc test). Figure 5B depicts the results of quantifying NADH levels in untreated Hela-ρ⁰ cells (untreated) and in Hela-ρ⁰ cells transfected with the mitoNEET-(RH)₉/pmtGFP complex or the mitoNEET-(RH)₉/mtDNA complex. "mtDNA" indicates cells transfected once with the mitoNEET-(RH) ₉/mtDNA complex, and "mtDNA × 3" indicates cells transfected three times with the mitoNEET-(RH)₉/mtDNA complex. "pmtGFP" indicates cells transfected once with the mitoNEET-(RH)₉/pmtGFP complex, and "pmtGFP × 3" indicates cells transfected three times with the mitoNEET-(RH)₉/pmtGFP complex. Error bars indicate standard error (SEM). "*" and "**" indicate p<0.05 and p<0.01, respectively (one way ANOVA with Dunnett's post-hoc test compared to untreated Hela-ρ⁰ cells).

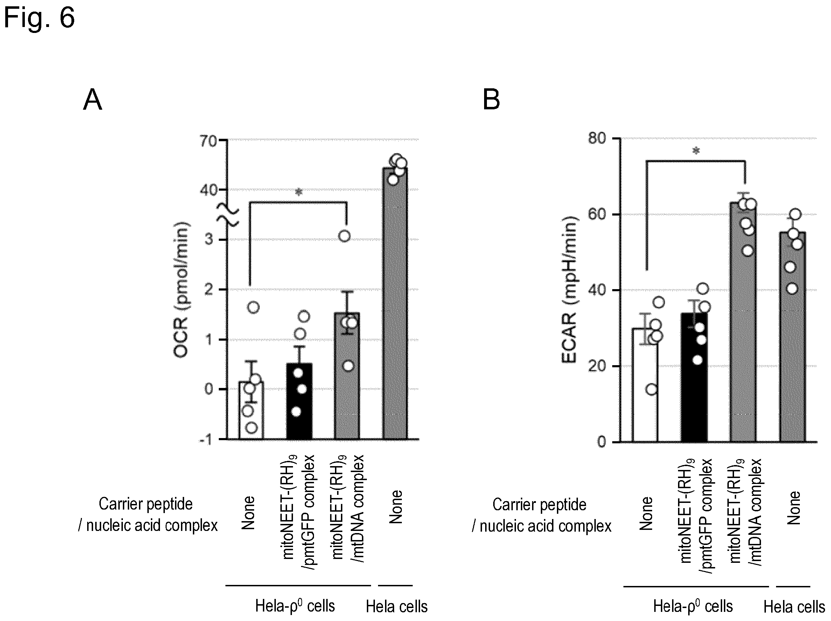

[Figure 6] Figure 6 depicts the results of measuring oxygen consumption rate (OCR) and extracellular acidification rate (ECAR). Figure 6A depicts the results of measuring the oxygen consumption rate (OCR) in untreated Hela-ρ⁰ cells, Hela-ρ⁰ cells transfected with the mitoNEET-(RH)₉/pmtGFP complex or the mitoNEET-(RH)₉/mtDNA complex, and Hela cells without mitochondrial defects. Figure 6B depicts the results of measuring extracellular acidification rate (ECAR) in untreated Hela-ρ⁰ cells, Hela-ρ⁰ cells transfected with the mitoNEET-(RH)₉/pmtGFP complex or the mitoNEET-(RH)₉/mtDNA complex, and Hela cells without mitochondrial defects. Error bars indicate standard error (SEM). "*" indicates p<0.05 (one way ANOVA with Dunnett's post-hoc test compared to untreated Hela-ρ⁰ cells).

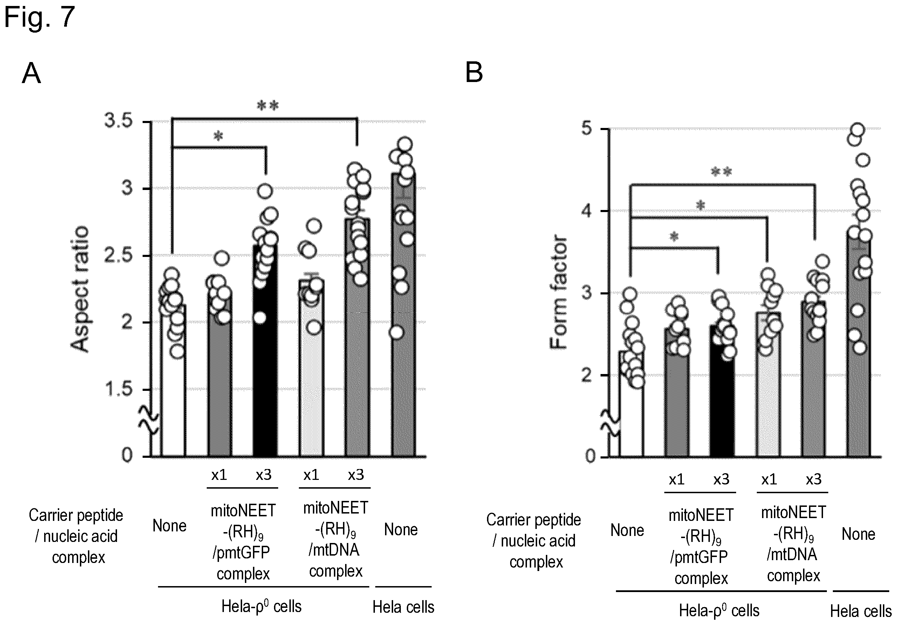

[Figure 7] Figure 7 depicts the results of evaluating mitochondrial morphology. Figure 7A depicts the aspect ratio (AR) of untreated Hela-ρ⁰ cells, Hela-ρ⁰ cells transfected with the mitoNEET-(RH)₉/pmtGFP complex or the mitoNEET-(RH)₉/mtDNA complex, and Hela cells without mitochondrial defects. Figure 7B depicts the form factor (FF) of untreated Hela-ρ⁰ cells, Hela-ρ⁰ cells transfected with the mitoNEET-(RH)₉/pmtGFP complex or the mitoNEET-(RH)₉/mtDNA complex, and Hela cells without mitochondrial defects. "*" and "**" indicate p<0.05 and p<0.01, respectively (one way ANOVA with Dunnett's post-hoc test compared to untreated Hela-ρ⁰ cells).

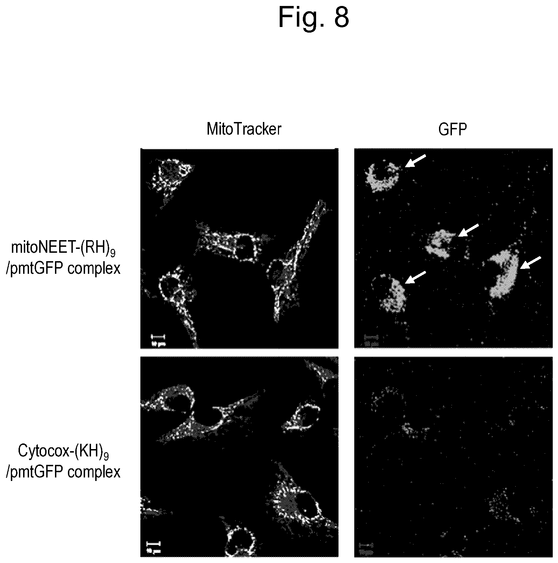

[Figure 8] Figure 8 depicts mitochondrial staining images and the fluorescent images of GFP in Hela cells transfected with the mitoNEET-(RH)₉/pmtGFP complex and Hela cells transfected with a Cytocox-(KH)₉/pmtGFP complex. Arrows indicate GFP expression.

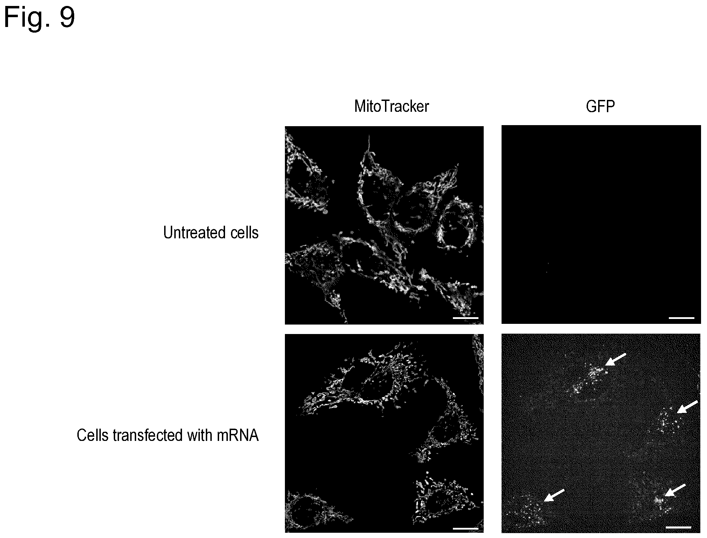

[Figure 9] Figure 9 depicts mitochondrial staining images and fluorescence images of GFP in HeLa cells transfected with mitoNEET-(RH)₉/mRNA complex and untreated cells. Arrows indicate GFP expression. Scale bar indicates 10 µm.

### Description of Embodiments

### 1. Carrier peptide

### 1-1. Overview

A first aspect of the present invention is a carrier peptide for introducing a nucleic acid to mitochondria. The carrier peptide of this aspect comprises a mitochondrial-targeting sequence and a polycationic sequence. The carrier peptide of this aspect binds to a nucleic acid with the polycationic sequence and can transfer the nucleic acid to the mitochondria based on the mitochondrial-targeting sequence.

### 1-2. Definition

The following terms, which are frequently used herein, are defined.

As used herein, "carrier peptide" refers to a peptide that forms a carrier peptide / nucleic acid complex (hereinafter also simply referred to as "complex") through interaction with a nucleic acid and can function as a transport unit that mediates the transfer of the nucleic acid to the mitochondria. The mode of interaction between the carrier peptide and the nucleic acid can be mainly electrostatic interaction and hydrogen bonding.

As used herein, "mitochondrial protein" means any protein which can be found in mitochondria. While more than 1,000 protein types have been found in mitochondria, only 37 gene types are encoded on human mitochondrial DNA, and the majority of mitochondrial proteins are encoded in a nuclear genome. Mitochondrial proteins encoded in the nuclear genome must be translated and then transferred from the cytoplasm to the mitochondria, but the mitochondrial proteins before transfer to the mitochondria are called preproteins. The preprotein can be transferred to mitochondria through recognition by receptors on the mitochondrial surface. In the mitochondrial outer membrane, Tom proteins such as Tom20, Tom22, and Tom70 are known to function as receptors for the transfer of different classes of preproteins.

As used herein, "mitochondrial-targeting sequence (MTS)" means an amino acid sequence that enables the transfer of a protein comprising MTS as a part of the amino acid sequence to mitochondria. The mitochondrial-targeting sequence may be a naturally occurring or artificial sequence, but in the case of a naturally occurring sequence, it is usually contained on the N-terminal side in a polypeptide. In many cases, the mitochondrial-targeting sequence is approximately 15 to 70 amino acids in length and is rich in positively charged basic amino acid residues. Examples of a protein comprising the mitochondrial-targeting sequence include a mitoNEET protein and yeast cytochrome c oxidase subunit IV (Cytocox), discussed below.

As used herein, "mitochondrial DNA (mtDNA)" means a circular DNA molecule contained in mitochondria. In the case of humans, 37 types of genes are encoded on mitochondrial DNA, comprising 22 types of tRNA genes, 2 types of rRNA genes, and 13 types of protein-coding genes. Mitochondrial DNA is sometimes referred to as a mitochondrial genome.

The "mitoNEET protein" refers to a mitochondrial protein that localizes to the mitochondrial outer membrane and can be involved in sensing reactive oxygen species (ROS). The mitoNEET protein is known to be a membrane-anchored protein comprising an N-terminal transmembrane domain, and a domain referred to as a CDGSH-type zinc finger (Wiley S. E. et al., Proc Natl Acad Sci U.S.A., 2007,104(13):5318-23.). A specific example of the mitoNEET protein is a human-derived mitoNEET (human mitoNEET) protein comprising or consisting of the amino acid sequence shown in SEQ ID NO: 17. Further examples of the mitoNEET protein also include a mitoNEET protein variant having activity functionally equivalent to that of the mitoNEET protein shown in the SEQ ID NO: 17 and mitoNEET orthologs of other biological species. Specifically, examples thereof include: a mitoNEET protein having an amino acid sequence derived from the amino acid sequence shown in the SEQ ID NO: 17 by the deletion, substitution, or addition of one or several amino acids; or a mitoNEET protein having 80% or more, 90% or more, 95% or more, 97% or more, 98% or more, or 99% or more amino acid identity to the amino acid sequence shown in the SEQ ID NO: 17. Specific examples of the mitoNEET ortholog include the mouse mitoNEET ortholog (SEQ ID NO: 20), the bovine mitoNEET ortholog (SEQ ID NO: 23), the duck (avian) mitoNEET ortholog (SEQ ID NO: 26), the salmon mitoNEET ortholog (SEQ ID NO: 29), the Drosophila mitoNEET ortholog (SEQ ID NO: 32), the zebrafish mitoNEET ortholog (SEQ ID NO: 35), the nematode (*C. elegans*) mitoNEET ortholog (SEQ ID NO: 38), and the *Arabidopsis thaliana* mitoNEET ortholog (SEQ ID NO: 41).

As used herein, "several" refers to, for example, 2 to 10, 2 to 7, 2 to 5, 2 to 4, or 2 to 3.

As used herein, "amino acid identity" refers to the percentage (%) of identical amino acid residues between two amino acid sequences relative to the total amino acid residues when the two amino acid sequences are aligned (alignment), and then gaps are introduced as necessary to achieve the highest degree of amino acid agreement between the two. Amino acid identity can be calculated using the BLAST or FASTA protein search system.

The mitoNEET protein is encoded by a mitoNEET gene. Specific examples of the mitoNEET gene include the human mitoNEET gene (for example, a gene comprising or consisting of the nucleotide sequence shown in the SEQ ID NO: 18) that encodes the human mitoNEET protein comprising the amino acid sequence shown in the SEQ ID NO: 17, and mitoNEET genes encoding the above mitoNEET variants or the mitoNEET orthologs (for example, any of the mitoNEET orthologs mentioned above) of other biological species. Specific examples thereof include: a mitoNEET gene having a nucleotide sequence derived from the nucleotide sequence shown in SEQ ID NO: 18 by the deletion, substitution, or addition of one or several nucleotides; or a mitoNEET gene having 80% or more, 90% or more, 95% or more, 97% or more, 98% or more, or 99% or more nucleotide identity to the nucleotide sequence shown in the SEQ ID NO: 18.

As used herein, "nucleotide identity" refers to the percentage (%) of identical nucleotides between two nucleotide sequences relative to the total nucleotides of the gene when the two nucleotide sequences are aligned (alignment) and gaps are introduced as necessary to achieve the highest degree of nucleotide agreement between the two.

### 1-3. Configuration

The carrier peptide of the present invention comprises a mitochondrial-targeting sequence and a polycationic sequence. In the carrier peptide of the present invention, the mitochondrial-targeting sequence consists of the N-terminal region comprising a transmembrane domain in the mitoNEET protein.

As used herein, the "transmembrane domain" of the mitoNEET protein refers to a domain that allows the mitoNEET protein to be stably incorporated into the membrane. In general, the transmembrane domain consists of a sequence of about 20 amino acids long, rich in hydrophobic amino acid residues, and can be easily predicted based on the amino acid sequence of a protein. It is usually predicted using transmembrane region prediction tools available in the art. Specific examples of the transmembrane domain of the mitoNEET protein can include the amino acid sequence IAAVTIAAGTAAIGYLAYK (SEQ ID NO: 1) located at positions 14 to 32 in the human mitoNEET protein consisting of the amino acid sequence shown in SEQ ID NO: 17, and a region corresponding to positions 14 to 32 of SEQ ID NO: 17 in the orthologs of other biological species of the mitoNEET protein. Specific examples of the region corresponding to positions 14 to 32 of SEQ ID NO: 17 in orthologs of other biological species can include the amino acid sequences shown in SEQ ID NO: 21, 24, 27, 30, 33, 36, 39, and 42.

In the carrier peptide of the present invention, the mitochondrial-targeting sequence consists of the N-terminal region comprising a transmembrane domain in the mitoNEET protein. As used herein, "N-terminal region comprising a transmembrane domain in the mitoNEET protein" means a region comprising the N-terminal amino acid residue to the transmembrane domain of the mitoNEET protein. The position of the C-terminal residue in the region comprising the N-terminal amino acid residue to the transmembrane domain is not limited, and such a region may be a region comprising up to the CDGSH domain or the full-length sequence of the mitoNEET protein. Examples of the N-terminal region include a region consisting of positions 1 to 32 or comprising positions 1 to 32 in the human mitoNEET protein consisting of the amino acid sequence shown in the SEQ ID NO: 17, a region corresponding to positions 1 to 32 of SEQ ID NO: 17 in the orthologs of other biological species of the mitoNEET protein, or a region comprising a sequence corresponding to positions 1 to 32 of SEQ ID NO: 17. In the human mitoNEET protein consisting of the amino acid sequence shown in SEQ ID NO: 17, the region consisting of positions 1 to 32 is MSLTSSSSVRVEWIAAVTIAAGTAAIGYLAYK (SEQ ID NO: 2). Specific examples of the region corresponding to positions 1 to 32 of SEQ ID NO: 17 in the orthologs of other biological species of the mitoNEET protein can include the amino acid sequences shown in the SEQ ID NO: 22, 25, 28, 31, 34, 37, 40, and 43. The sequence of the mitoNEET protein derived from the same biological species from which a cell to which the carrier peptide / nucleic acid complex is introduced is derived, as described below, or a closely related species, is preferred because high mitochondrial delivery activity can be produced.

In the carrier peptide of the present invention, two or more types of the mitochondrial-targeting sequences may be used in combination as the mitochondrial-targeting sequence.

As used herein, "polycationic sequence" means an amino acid sequence comprising a plurality of amino acid residues that can be positively charged. Specifically, it is a peptide sequence that comprises at least three cationic amino acid residues and forms a stable bond by electrostatic interaction with a negatively charged nucleic acid under physiological conditions.

As used herein, "cationic amino acid (residue)" means an amino acid (residue) that can be positively charged under physiological pH or near-physiological pH conditions. Cationic amino acids may be either standard or non-standard amino acids. Examples of standard amino acids include lysine (K), arginine (R), and histidine (H). Examples of non-standard amino acids include, in addition to lysine (K) derivatives, arginine (R) derivatives, and histidine (H) derivatives, basic amino acids such as ornithine and their derivatives, and amino acid derivatives in which cationic groups are introduced into acidic amino acids (e.g. aspartic acid, glutamic acid).

As used herein, "derivative" refers to a compound with a similar structure having the same or similar basic structure and properties. Examples of the derivatives include compounds comprising substituents, biosynthetic intermediates, and metabolites. Those skilled in the art can determine whether or not a compound is a derivative of another compound based on technical common sense. Specific examples of amino acid derivatives can include: a structure in which α-carbon is alkylated (e.g., methylated); a structure in which the C-terminus of an amino acid is amidated (e.g., a structure amidated by alkylamine such as ethylamine, ethanolamine, or ethylenediamine); and a structure in which an amino acid side chain is modified (e.g., a structure in which ε-amino group of lysine is acylated).

In one embodiment, the polycationic sequence is a peptide sequence that comprises at least three amino acid residues selected from lysine (K) residues, arginine (R) residues, histidine (H) residues, and derivatives of any of them, and forms a stable bond by electrostatic interaction with a negatively charged nucleic acid under physiological conditions. Lysine residues, arginine residues, and derivatives of any of them that are positively charged under physiological conditions are particularly preferred.

In addition to the cationic amino acid residues described above, the polycationic sequence can also comprise a neutral amino acid under conditions such that their overall properties remain sufficiently cationic to form a stable bond with a protein under physiological conditions. This can be tested by a simple experiment in which a protein is added. For example, it is possible to test by mixing a carrier peptide with a target nucleic acid and measuring the zeta potential and/or particle size of the particles in the mixture. For example, by determining whether the zeta potential changes from a negative value to a positive value when a carrier peptide is added to a target nucleic acid, the presence or absence of the formation of a complex of the carrier peptide and the nucleic acid can be examined.

The polycationic sequence of a carrier peptide comprises at least three cationic amino acid residues, and the upper limit is not particularly limited. However, the length of the polycationic sequence ranges from preferably 5 to 100 amino acid residues, more preferably 10 to 50, and even more preferably 20 to 40 amino acid residues. The percentage of cationic amino acid residues in the polycationic sequence is preferably 40 mol% or more, more preferably 60 mol% or more, even more preferably 80 mol% or more, and most preferably 90 mol% or more. The polycationic sequence consisting solely of polycationic amino acid residues is most preferably used.

For example, the polycationic sequence comprises 4 or more, 5 or more, 6 or more, 7 or more, 8 or more, 10 or more, 15 or more, or 20 or more and/or 60 or less, 50 or less, 40 or less, or 35 or less cationic amino acid residues (e.g., lysine residues, arginine residues, histidine residues, or derivatives of any of them). The polycationic sequence may comprise, for example, 3 or more, 5 or more, 7 or more, or 9 or more consecutive lysine residues, arginine residues, histidine residues, and/or derivatives of any of them. The efficiency of introduction to mitochondria can be controlled by selecting the polycationic sequence adequately. Preferred examples of the polycationic sequence can include a sequence formed by repeating the amino acid sequence KH (for example, a sequence formed by repeating the amino acid sequence KH 3 to 20 times, 5 to 15 times, or 7 to 12 times, such as 9 to 10 times) and a sequence formed by repeating the amino acid sequence RH (for example, a sequence formed by repeating the amino acid sequence RH 3 to 20 times, 5 to 15 times, or 7 to 12 times, such as 9 to 10 times). Further examples of the polycationic sequence include a consecutive arginine (R) sequence (for example, a sequence of consecutive 3 to 20, 5 to 15, or 7 to 12 Rs), a consecutive lysine (K) sequence (for example, a sequence of consecutive 3 to 20, 5 to 15, or 7 to 12 Ks), or a consecutive histidine (H) sequence (for example, a sequence of consecutive 3 to 20, 5 to 15, or 7 to 12 Hs). Specific examples of the polycationic sequence can include RHRHRHRHRHRHRHRHRH (SEQ ID NO: 3), KHKHKHKHKHKHKHKHKH (SEQ ID NO: 4), and KKKKKKKK (SEQ ID NO: 19).

The carrier peptide of the present invention corresponds to a linear fusion of the mitochondrial-targeting sequence and the polycationic sequence. In this fusion, the polycationic sequence can be bound to the N-terminal and/or C-terminal side of the mitochondrial-targeting sequence, but preferably the polycationic sequence is bound to the C-terminal side of the mitochondrial-targeting sequence. In the carrier peptide of the present invention, the position of the mitochondrial-targeting sequence is not limited, but it is preferred to be placed on the N-terminal side of the carrier peptide for efficient delivery to mitochondria. One or two or more, one to a plural number of, or about one to three polycationic sequences described above may be bound to the mitochondrial-targeting sequence, and preferably one polycationic sequence can be bound to the mitochondrial-targeting sequence. A peptide with the polycationic sequence bound to the mitochondrial-targeting sequence can also be synthesized according to common peptide synthesis methods, such as solid-phase methods, for example, or can also be bioengineered using genetic recombination. Alternatively, a separately prepared mitochondrial-targeting sequence and a polycationic sequence can be chemically bound, for example, by cross-linking reactions. Upon binding of the mitochondrial-targeting sequence to a polycationic sequence, an oligopeptide linker or the like can be placed to mediate adequately between the two. For example, a linker consisting of one to a plurality of amino acids can be placed for mediation, and amino acid residues constituting the linker can be selected adequately. Since the mitochondrial-targeting sequence exhibits its properties prominently at the N-terminus, the mitochondrial-targeting sequence is preferably bound to the N-terminal side of the polycationic sequence. When the carrier peptide of the present invention is prepared by recombinant DNA technology, for example, a DNA fragment encoding a polycationic sequence is bound to one or both ends of a DNA fragment encoding a cell-penetrating sequence by a ligation reaction with an appropriate DNA adapter. Such genetic engineering methods are well known to those skilled in the art of molecular biology.

The carrier peptide of the present invention can further comprise any sequence in addition to the mitochondrial-targeting sequence and the polycationic sequence. For example, the carrier peptide may comprise a linker sequence between the mitochondrial-targeting sequence and the polycationic sequence. In addition, the carrier peptide may also comprise a cell-penetrating sequence for the purpose of increasing the efficiency of introduction to cells. As used herein, "cell-penetrating sequence" means the sequence of a cell-penetrating peptide (CPP). The cell-penetrating peptide means a peptide that can penetrate the cell membrane and enter the cell. Examples of the cell-penetrating peptide include, but are not limited to, BP100, HIV Tat (SEQ ID NO: 7), Tat₂, Penetratin, pVEC, pAntp, HSV-1 VP22, MAP (Model amphiphilic peptide), Transportan, R7, MPG, and Pep-1.

Examples of the sequence of the carrier peptide of the present invention include the amino acid sequence comprising MSLTSSSSVRVEWIAAVTIAAGTAAIGYLAYKRHRHRHRHRHRHRHRHRH (SEQ ID NO: 5, mitoNEET-(RH)₉) or MSLTSSSSVRVEWIAAVTIAAGTAA IGYLAYKKHKHKHKHKHKHKHKHKH (SEQ ID NO: 6, mitoNEET-(KH)₉).

### 1-4. Effects

The carrier peptide of the present invention is mixed with a nucleic acid to form a carrier peptide / nucleic acid complex, so that the nucleic acid can be transferred to mitochondria. A gene encoded by the nucleic acid can be expressed in the mitochondria. For example, the carrier peptide of the present invention is mixed with mitochondrial DNA (mtDNA), so that a carrier peptide/mtDNA complex can be formed, and thus mitochondrial function can be restored based on the mtDNA introduced by the complex.

The carrier peptide of the present invention comprises a mitochondrial-targeting sequence derived from the mitoNEET protein and is dramatically more efficient at introducing nucleic acids to mitochondria than the carrier peptide comprising a mitochondrial-targeting sequence derived from yeast cytochrome c oxidase subunit IV (Cytocox).

According to the carrier peptide of the present invention, a reagent for introducing a target nucleic acid to mitochondria comprising or consisting of the carrier peptide of the present invention is also provided. The reagent for introducing can form a carrier peptide / nucleic acid complex by electrostatic interaction with a target nucleic acid.

According to the carrier peptide of the present invention, a kit for introducing a target nucleic acid to a target cell is also provided. The kit comprises the carrier peptide of the present invention or the above-mentioned reagent for introducing. The kit may comprise instruction manuals, reagents for forming complexes and introducing to cells, instruments and the like.

### 2. Nucleic acid encoding carrier peptide

### 2-1. Overview

A second aspect of the present invention is a nucleic acid encoding a carrier peptide.

### 2-2. Configuration

The "nucleic acid encoding carrier peptide" of the present invention may be a nucleic acid that encodes any of the carrier peptides described in the first aspect. The nucleotide sequence of such nucleic acid is not limited. Examples thereof include codon-optimized nucleotide sequences and nucleotide sequences with an additional start codon (ATG) added to the 5' end side.

Examples of the nucleic acid of the present invention include a gene expression vector comprising a nucleic acid encoding a carrier peptide in an expressible form. The gene expression vector is a gene expression vector that comprises a nucleic acid encoding a carrier peptide and a promoter and is capable of expressing the carrier peptide in a cell. The gene expression vector may comprise, in addition to nucleic acids and promoters, which are said components, components such as drug resistance genes, introns, enhancers, terminators, replication origins, and/or poly A signals, as needed.

As used herein, "gene expression vector" refers to a vector comprising a gene or gene fragment (hereinafter denoted as "gene, etc.") in an expressible form and includes an expression unit that can control the expression of the gene, etc. The gene expression vector may be a plasmid vector or a viral vector. The plasmid vector may be a commercially available expression vector for mammalian cells, such as a pSI vector (PROmega), or a replicable shuttle vector between mammalian cells and bacteria such as *E. coli.*

As used herein, "in an expressible form" refers to that a gene or the like to be expressed is placed in the downstream region of a promoter under the control of the promoter. Plasmid vectors, viral vectors, and the like are known as vectors, and any of these vectors can be used. Usually, the vector may be a plasmid vector that can be easily genetically recombined.

As used herein, "promoter" refers to a gene expression regulatory region that can control the expression of a gene or the like placed downstream (3' end side) in a cell into which a gene expression vector is introduced. The promoter may be either a constitutively active promoter or an expression-inducible promoter.

According to the present invention, host cells transfected with the nucleic acid of this aspect are also provided.

### 3. Carrier peptide / nucleic acid complex

### 3-1. Overview

A third aspect of the present invention is a carrier peptide / nucleic acid complex. The carrier peptide / nucleic acid complex of this aspect comprises the carrier peptide of the first aspect and a nucleic acid to be introduced to mitochondria. The carrier peptide / nucleic acid complex of this aspect enables direct and efficient gene introduction to mitochondria.

### 3-2. Configuration

The carrier peptide / nucleic acid complex of this aspect comprises the carrier peptide of the first aspect and a nucleic acid.

The type of a nucleic acid contained in the carrier peptide / nucleic acid complex is not particularly limited. For example, the nucleic acid may be DNA, RNA, or a DNA-RNA hybrid, and linear or cyclic. The nucleic acid may also be single or double stranded. DNA may comprise a gene encoding a protein or RNA and may be, for example, cDNA, a DNA vector such as plasmid DNA, genomic DNA or a fragment thereof, or mitochondrial DNA (mtDNA) or a fragment thereof. RNA may be protein-coding mRNA or non-coding RNA such as rRNA, tRNA (e.g., mitochondrial tRNA), miRNA, or siRNA. Furthermore, the nucleic acid may be a nucleic acid that does not encode a protein or RNA, and may be, for example, a nucleic acid aptamer or an antisense nucleic acid.

Chemical modification can also be made to a nucleic acid contained in the carrier peptide / nucleic acid complex, as long as the negative charge of the phosphate backbone that mediates ionic bonding to the polycationic sequence of the carrier peptide is preserved. Examples of suitably modified nucleic acids can include thioates and dithioates. In addition, nucleic acids with chemically modified nucleotide bases can also be used.

The nucleic acid contained in the carrier peptide / nucleic acid complex can contain genetic information for expression in mitochondria. The type of a protein of interest (hereinafter also simply referred to as "target protein") to be encoded by the nucleic acid and expressed in mitochondria or the type of RNA of interest (hereinafter also simply referred to as "target RNA") to be expressed in mitochondria is not limited. The target protein may be, for example, any of structural proteins, secreted proteins, enzymes, antibodies, labeled proteins, regulatory proteins, selection marker proteins, and the like. Examples of target RNA can include mitochondrial tRNA and mitochondrial rRNA. Two (2) or more, 3 or more, 4 or more, or 5 or more target proteins and/or target RNAs can also be introduced to mitochondria simultaneously.

In one embodiment, the target protein is a mitochondrial protein. The mitochondrial protein may be encoded by nuclear genomic DNA or mitochondrial DNA. For example, the target protein may be a mitochondrial protein that can be involved in energy production (for example, oxidative phosphorylation) in mitochondria, control of reactive oxygen species, or gene expression, etc., in mitochondria. Specific examples of such mitochondrial proteins include NADH reductase subunits (e.g., ND1 to ND6 proteins), cytochrome b, cytochrome oxidase (e.g., COI to COIII), ATPase (e.g., ATPase 6 or ATPase 8), cytochrome c, and taurine modification enzyme Mto1. Preferred mitochondrial proteins are any proteins encoded by mitochondrial DNA.

The target RNA may also be mitochondrial tRNA (mt-tRNA) or mitochondrial rRNA. Specific examples of mt-RNA can include mt-tRNA^{Leu(UUR)}, mt-tRNA^{Trp}, mt-tRNA^{Lys}, mt-tRNA^{Glu}, and mt-tRNA^{Gln}. Mitochondrial rRNA can be, for example, 12S rRNA or 16S rRNA.

Further examples of the target protein include genome-editing proteins. As used herein, "genome editing" refers to specific cutting and editing of target sites on the genome, for example, knocking in or knocking out specific genes with respect to wild-type genomic genes. Examples of genome editing proteins include TALEN (transcription activator-like effector nuclease), Cas9 (CRISPR associated protein 9), and ZFN (zinc finger nuclease), and preferably TALEN and Cas9.

When the genome editing protein is Cas9, guide RNA must be introduced to edit or modify the genome. Therefore, in this case, it is preferable that the complex comprises the guide RNA as the target RNA and is delivered into a cell together with a nucleic acid encoding the Cas9 protein, but it may be introduced into the cell by other techniques. Examples of such techniques include transfection with guide RNA or a vector such as a plasmid comprising guide RNA.

The carrier peptide / nucleic acid complex of the present invention is characterized in that a nucleic acid can be introduced to mitochondria without being limited by the type or size of the nucleic acid, and can use double-stranded DNA of as short as about 20 nucleotides long to several hundred kilobase pairs. For example, for a single-stranded nucleic acid, it may be 15 nucleotides long or longer, 1,000 nucleotides long or longer, 2,000 nucleotides long or longer, 4,000 nucleotides long or longer, 8,000 nucleotides long or longer, or 12,000 nucleotides long or longer, and/or 100,000 nucleotides long or shorter, 50,000 nucleotides long or shorter, 40,000 nucleotides long or shorter, 30,000 nucleotides long or shorter, 20,000 nucleotides long or shorter, 19,000 nucleotides long or shorter, 18,000 nucleotides long or shorter, 17,000 nucleotides long or shorter, 16,000 nucleotides long or shorter, 15,000 nucleotides long or shorter, 14,000 nucleotides long or shorter, or 13,000 nucleotides long or shorter. For double-stranded nucleic acids (for example, double-stranded DNA molecules), it may be 15 bp or more, 1 kbp or more, 2 kbp or more, 4 kbp or more, 8 kbp or more, or 12 kbp or more, and/or 100 kbp or less, 50 kbp or less, 40 kbp or less, 30 kbp or less, 20 kbp or less, 19 kbp or less, 18 kbp or less, 17 kbp or less, 16 kbp or less, 15 kbp or less, 14 kbp or less, or 13 kbp or less, for example, 1 kbp to 20 kbp, or 4 kbp to 16 kbp.

The form of the complex of the carrier peptide and the nucleic acid is not limited, but is usually in the form of particles, and the average hydrodynamic diameter of which is, for example, 10 nm or more, 20 nm or more, 30 nm or more, 40 nm or more, 50 nm or more, 100 nm or more, 150 nm or more, or 200 nm or more, and/or 700 nm or less, 600 nm or less, 500 nm or less, 400 nm or less, or 300 nm or less. Preferred particle sizes are 20 nm to 120 nm or 40 nm to 100 nm. The average hydrodynamic diameter can be measured by the dynamic light scattering (DLS) method. The present inventors have discovered that a complex with such average hydrodynamic diameter can be transferred to mitochondria.

In the carrier peptide / nucleic acid complex of this aspect, the number of amino and guanidino groups derived from the carrier peptide/the number of phosphate groups derived from the nucleic acid (N/P ratio) is not particularly limited, as long as the efficiency of complex formation is not impaired, and may be the ratio such that particles can be formed in the above average hydrodynamic diameter range, for example. For example, the N/P ratio is 0.2 or more, 0.5 or more, 0.7 or more, 0.9 or more, or 1.0 or more, and/or 100 or less, 50 or less, 40 or less, 30 or less, 20 or less, 15 or less, 10 or less, 8 or less, 6 or less, 4 or less, 3 or less, or 2 or less, for example 0.2 or more to 100 or less. In the case of a complex of a cationic polymer and a nucleic acid, there is a known example of such a complex formed at an N/P ratio of 16 or 32, and this is similarly applicable to the present invention.

### 3-3. Effects

The carrier peptide / nucleic acid complex of the present invention is transferred to the mitochondria in cells, and the gene encoded by the nucleic acid contained in the carrier peptide / nucleic acid complex is expressed in the mitochondria. The transfer pathway of the carrier peptide / nucleic acid complex is not limited, but the complex can be transferred, for example, via endosomes/lysosomes to the mitochondria.

According to the carrier peptide / nucleic acid complex of the present invention, there is no upper limit on the size of a nucleic acid to be delivered, and mtDNA can also be introduced to mitochondria.

### 4. Pharmaceutical composition for treating mitochondrial disease

### 4-1. Overview

A fourth aspect of the present invention is a pharmaceutical composition for treating a mitochondrial disease. The pharmaceutical composition for treating a mitochondrial disease of the present invention comprises the carrier peptide / nucleic acid complex of the third aspect, and can treat a mitochondrial disease.

### 4-2. Configuration

The pharmaceutical composition of the present invention comprises an active ingredient as an essential constituent component, and a pharmacologically acceptable carrier or other drugs as optional components. The pharmaceutical composition of the present invention can also be composed of only the active ingredient. However, in order to facilitate the formation of a dosage form and to maintain the pharmacological effect and/or dosage form of the active ingredient, the pharmaceutical composition is preferably composed of a pharmacologically acceptable carrier described later.

### 4-2-1. Constituent components

Each component constituting the pharmaceutical composition of the present invention will be specifically described below.

### (1) Active ingredient

The active ingredient in the pharmaceutical composition of the present invention is the carrier peptide / nucleic acid complex of the present invention. The carrier peptide and the nucleic acid constituting the complex are composed as described above, and therefore the description will be omitted. The number of the carrier peptide / nucleic acid complex contained in the pharmaceutical composition of the present invention is not limited, and may be one or more.

### (2) Pharmacologically acceptable carrier

The "pharmacologically acceptable carrier" refers to a solvent and/or an additive that can be commonly used in the field of pharmaceutical formulation technology and that are little or not harmful to living organisms.

Examples of pharmacologically acceptable solvents include water, ethanol, propylene glycol, ethoxylated isostearyl alcohol, polyoxylated isostearyl alcohol, and polyoxyethylene sorbitan fatty acid esters. These are preferably sterilized and, if necessary, adjusted to be isotonic with blood.

Furthermore, examples of pharmacologically acceptable additives include excipients, binding agents, disintegrants, fillers, emulsifiers, addition agents for regulating flow, and lubricants.

Examples of excipients include sugars such as monosaccharides, disaccharides, cyclodextrins, and polysaccharides (more specific examples thereof include, but are not limited to, glucose, sucrose, lactose, raffinose, mannitol, sorbitol, inositol, dextrin, maltodextrin, starch, and cellulose), metal salts (for example, sodium chloride, sodium phosphate or calcium phosphate, calcium sulfate, magnesium sulfate, calcium carbonate), citric acid, tartaric acid, glycine, low, medium, or high molecular weight polyethylene glycols (PEGs), pluronics, kaolin, and silicic acid, or combinations thereof.

Examples of binding agents include starch paste made from corn, wheat, rice, or potato starch, simple syrup, glucose solution, gelatin, tragacanth, methylcellulose, hydroxypropylmethylcellulose, sodium carboxymethylcellulose, shellac, and/or polyvinylpyrrolidone.

Examples of disintegrants include said starches, lactose, carboxymethyl starch, crosslinked polyvinylpyrrolidone, agar, laminaran powder, sodium hydrogen carbonate, calcium carbonate, alginic acid or sodium alginate, polyoxyethylene sorbitan fatty acid esters, sodium lauryl sulfate, and monoglyceride stearate, or salts thereof.

Examples of fillers include said sugars and/or calcium phosphates (e.g., tricalcium phosphate or calcium hydrogen phosphate).

Examples of emulsifiers include sorbitan fatty acid esters, glycerin fatty acid esters, sucrose fatty acid esters, and propylene glycol fatty acid esters.

Examples of addition agents for regulating flow and lubricants include silicates, talc, stearate, or polyethylene glycol.

In addition to the above additives, other additives can also be contained as needed, such as corrigents, dissolution aids (solubilizers), suspending agents, diluents, surfactants, stabilizers, agents for accelerating absorption (e.g., quaternary ammonium salts, sodium lauryl sulfate), agents for bulking, agents for retaining moisture (e.g., glycerin, starch), adsorbents (e.g., starch, lactose, kaolin, bentonite, colloidal silicate), agents for inhibiting disintegration (e.g., white sugar, stearin, cocoa butter, hydrogenated oil), coating agents, coloring agents, preservatives, antioxidants, perfume, flavor agents, sweeteners, and buffering agents.

### (3) Other agents

The pharmaceutical composition of the present invention can also comprise other agents within the scope of not losing the pharmacological effects of the above active ingredients. Here, examples of "other agents" include other agents for treating mitochondrial diseases. The agent may be an agent having a pharmacologic effect unrelated to the direct therapeutic effect on mitochondrial diseases. An example thereof is an agent for protecting gastric mucosa.

### 4-2-2. Dosage form

The dosage form of the pharmaceutical composition of the present invention is not particularly limited as long as it does not or hardly inactivate the carrier peptide / nucleic acid complex of the present invention, which is an active ingredient, and can fully exert its pharmacological effect *in vivo* after administration.

Dosage forms can be classified into liquid dosage forms or solid dosage forms depending on their form, but the pharmaceutical composition of the present invention may be either of them. Dosage forms can also be broadly classified into oral dosage forms and parenteral dosage forms depending on the method of administration, but either of these may be used.

Specific examples of dosage forms include, in the case of oral dosage forms, liquid dosage forms such as suspensions, emulsions, and syrups, and in the case of solid dosage forms, powders (including powders, powder agents, and candy powders), granules, tablets, capsules, sublingual tablets, and troches. Examples of parenteral dosage forms include liquid dosage forms such as injections, suspensions, and emulsions, and solid dosage forms such as creams, ointments, plasters, patches, and suppositories. Preferred dosage forms are any of the oral dosage forms, or, in the case of parenteral dosage forms, liquid dosage forms such as injections.

### 4-2-3. Method of administration

All methods known in the art can be applied to the pharmaceutical composition of the present invention, as long as they are methods that enable the administration of an effective amount of the carrier peptide / nucleic acid complex of the present invention, which is an active ingredient, to a living body for treating a mitochondrial disease.

As used herein, "effective amount" refers to the amount of the active ingredient required to exert its function, i.e., the amount required for the pharmaceutical composition of the present invention to treat a mitochondrial disease and the amount that has little or no harmful side effects on the living body to which it is applied. This effective amount may vary depending on conditions such as the information of a subject, the route of administration, and the frequency of administration. "Subject" or "target" refers to an individual animal to which the pharmaceutical composition of the present invention is applied. The preferred subject is a human. "The information of a subject" refers to various kinds of information of an individual subject, including, for example, the subject's age, body weight, sex, general health condition, drug sensitivity, and the presence or absence of medications being taken. The effective amount and the dosage calculated based on it are determined by the judgment of a physician or veterinarian depending on the information etc., of each individual subject. When it is necessary to administer a large amount of the pharmaceutical composition of the present invention to obtain a sufficient effect in treating or preventing enterococcal infection, the pharmaceutical composition can be administered in several divided doses to reduce the burden on the subject.

The pharmaceutical composition of the present invention may be administered either systemically or locally. Examples of systemic administration include intravascular injection such as intravenous injection and oral administration. Examples of local administration include rectal administration and intraperitoneal administration. A preferred administration method is oral administration, rectal administration, intraperitoneal administration, or intravenous administration.

When the pharmaceutical composition of the present invention is administered or ingested, the dosage or intake is appropriately selected depending on the age, body weight, symptoms, and health condition of the subject, the type of a composition (a pharmaceutical product, food and drink, etc.), and the like. For example, the dosage or intake may be 0.001 mg/kg/day to 1,000 mg/kg/day, 0.01 mg/kg/day to 500 mg/kg/day, 0.1 mg/kg/day to 200 mg/kg/day, 1 mg/kg/day to 100 mg/kg/day, 5 mg/kg/day to 50 mg/kg/day, or 10 mg/kg/day.

### 4-2-4. Target disease

As used herein, "mitochondrial disease(s)" is not particularly limited as long as it is a disease related to mitochondrial hypofunction or dysfunction. The mitochondrial disease may be caused by a mutation in mtDNA, or caused by a mutation in nuclear genomic DNA. This is because even if the mitochondrial abnormality is caused by a mutation in nuclear genomic DNA, the mitochondrial abnormality can be improved by causing the expression of a normal protein, in which the mutation has been repaired, in mitochondria. In addition, the mitochondrial disease may be hereditary or non-hereditary. For example, the mitochondrial disease may be caused by heteroplasmy that can occur due to aging. Specific examples of the mitochondrial disease can include, but are not limited to, mitochondrial myopathy, encephalopathy, lactic acidosis, and stroke (MELAS) syndrome, pyruvate dehydrogenase deficiency disease, myoclonic epilepsy and ragged red fibers (MERRF) syndrome, chronic progressive external ophthalmoplegia (CPEO) syndrome, Leigh encephalopathy, Leber's hereditary optic neuropathy (LHON), Kearns-Sayre syndrome (KSS), Pearson syndrome (PS), Leber's hereditary optic neuropathy (LHON), dominant optic atrophy (DOA), and Friedreich's ataxia (FRDA), as well as sensorineural hearing loss, Alzheimer's disease, and Parkinson's disease. In sensorineural hearing loss, Alzheimer's disease, and Parkinson's disease, mtDNA mutations can be detected in pathological tissues.

Mitochondrial diseases are known to be prone to express symptoms in the nerves, muscles, and heart, etc., which require a relatively large amount of energy. Mitochondrial diseases can usually be diagnosed based on brain disorders such as convulsions, strokes, mental symptoms, and developmental delays, sensory disorders such as difficulty in seeing objects and hearing sounds, and muscle weakness such as inability to exercise and easy fatigue, but it is also possible to detect mitochondrial diseases by detecting mutations in the mtDNA of test samples derived from subjects, such as biopsy samples.

### 4-3. Effects

By administering the pharmaceutical composition of the present invention to a patient with a mitochondrial disease, the carrier peptide / nucleic acid complex, which is the active ingredient, can be incorporated into cells and transferred to mitochondria. For example, it is possible to restore mitochondrial hypofunction or dysfunction by the expression of a gene encoded by a nucleic acid contained in the carrier peptide / nucleic acid complex in mitochondria.

When the pharmaceutical composition of the present invention comprises mtDNA as a nucleic acid, the heteroplasmy can be improved by increasing the copy number of normal mtDNA.

The pharmaceutical composition for treating a mitochondrial disease of the present invention can also be used for preventing the mitochondrial disease. In this case, the pharmaceutical composition of the present invention can be administered to a subject before the onset of a mitochondrial disease as a pharmaceutical composition for preventing the mitochondrial disease.

The present invention also provides a method for treating or preventing a mitochondrial disease, comprising the step of administering the carrier peptide / nucleic acid complex or pharmaceutical composition of the present invention to a subject, such as a patient with a mitochondrial disease. The use of the carrier peptide / nucleic acid complex or pharmaceutical composition of the present invention is also provided in the production of a medicine for treating or preventing a mitochondrial disease.

### 5. Method for producing carrier peptide / nucleic acid complex

### 5-1. Overview

A fifth aspect of the present invention is a method for producing a carrier peptide / nucleic acid complex. With the production method of this aspect, nano-sized particles that are highly efficient at introducing to mitochondria can be efficiently prepared.

### 5-2. Method

The production method of the present invention comprises a complex formation step as an essential step.

In the production method of the present invention, the "complex formation step" refers to a step of mixing the carrier peptide of the first aspect with a nucleic acid to form a carrier peptide / nucleic acid complex comprising the carrier peptide and the nucleic acid. In this aspect, the carrier peptide, the nucleic acid, and the carrier peptide/protein complex are composed as described above, and therefore the description will be omitted.

For the carrier peptide and nucleic acid to be mixed in this step, the number of amino groups and guanidino groups derived from the carrier peptide/the number of phosphate groups derived from the nucleic acid (N/P ratio) can be used under conditions that allow efficient complex formation. The N/P ratio can be, for example, 0.2 or more and 20 or less, and may be 0.2 or more, 0.5 or more, 0.7 or more, 0.9 or more, or 1.0 or more, and/or 20 or less, 15 or less, 10 or less, 8 or less, 6 or less, 4 or less, 3 or less, or 2 or less, for example, 0.2 or more and 10 or less. A preferred N/P ratio is 2 to 4 or 2.5 to 3.

The molar ratio of the carrier peptide to the nucleic acid is, for example, 1 or more, 2 or more, 3 or more, 4 or more, or 5 or more, and/or 40 or less, 30 or less, 25 or less, 20 or less, 15 or less, or 10 or less.

The step of mixing the carrier peptide with the nucleic acid to form a complex can be carried out, for example, by mixing the carrier peptide with the nucleic acid in a solution. In this case, the concentration of the carrier peptide is usually 10 µg/mL to 10 mg/mL, preferably 100 µg/mL to 1 mg/mL, and the concentration of the carrier peptide solution is usually 1 µg/mL to 10 mg/mL, preferably 10 µg/mL to 1 mg/mL.

The conditions for the step of mixing the carrier peptide with the nucleic acid to form a complex are not particularly limited, but the step can be performed, for example, by incubating at ordinary temperature (20°C to 35°C) for a few minutes to several hours, for example 5 minutes to 6 hours, 10 minutes to 3 hours, and preferably 20 minutes to 1 hour.

The complex formed in this step can be purified by centrifugation, sedimentation, filtration, and the like, as necessary. Purifying the complex makes it possible to remove carrier peptides and nucleic acids that did not form any complex.

The average hydrodynamic diameter of the complex formed by the production method of the present invention is, for example, 100 nm or more, 150 nm or more, or 200 nm or more, and/or 700 nm or less, 600 nm or less, 500 nm or less, 400 nm or less, or 300 nm or less.

### 5-3. Effects

According to the production method of this aspect, the polycationic sequence contained in the carrier peptide binds to the nucleic acid based on electrostatic interaction or the like to form a carrier peptide / nucleic acid complex.

### 6. Method of introducing nucleic acid

### 6-1. Overview

A sixth aspect of the present invention is a method of introducing a nucleic acid. By the method of introducing a nucleic acid of this aspect, a carrier peptide / nucleic acid complex can be introduced to mitochondria.

### 6-2. Method

The method of introducing a nucleic acid of the present invention comprises, as essential steps, a contact step of contacting the carrier peptide / nucleic acid complex of the third aspect with an isolated target cell, a transfer step of culturing the target cell after the contact step to transfer the carrier peptide / nucleic acid complex to mitochondria, and comprises, as an optional step, a complex formation step of forming a carrier peptide / nucleic acid complex. The complex formation step is the same as that of the fifth aspect described above, and therefore the description will be omitted.

### (Contact step)

The step of contacting the complex with a target cell can be performed by a method known in the art and is not particularly limited. For example, it can be performed by contacting a solution containing the carrier peptide / nucleic acid complex of the present invention with an isolated target cell and incubating the cell at ordinary temperature (20°C to 35°C). The incubation time is, for example, 1 minute to 150 hours, preferably 1 hour to 48 hours or 3 hours to 24 hours.

The target isolated cell to which the carrier peptide / nucleic acid complex is introduced may be a cell comprising mitochondria, and is not limited as long as it is a eukaryotic cell. Examples of eukaryotic cells include fungal cells (e.g., yeast cells), algae cells, plant cells, protozoan cells, insect cells, nematode cells, fish cells, avian cells (e.g., chicken cells), and mammalian cells (e.g., mouse cells, chimpanzee cells, and human cells). Eukaryotic cells are preferably mammalian cells. Specific examples of mammalian cells include, but are not limited to, CHO cells, COS cells, Vero cells, HEK293 cells and their derivatives, HeLa cells, NIH3T3 cells, K562 cells, ES cells, iPS cells, and neural stem cells. The cells may be derived from a patient with a mitochondrial disease or the like.

In this step, the method of introducing a double-stranded circular DNA vector into a host cell is not particularly limited. For example, the efficiency of introducing the complex can also be improved by referring to gene introduction methods (transformation methods) known in the art described in Green & Sambrook, 2012, Molecular Cloning: A Laboratory Manual Fourth Ed., Cold Spring Harbor Laboratory Press, Cold Spring Harbor, New York, etc. Specifically, lipofection, electroporation, microinjection, particle bombardment, and the like can be used.

### (Transfer step)

A method for culturing target cells after the contact step in the transfer step of transferring the carrier peptide / nucleic acid complex to mitochondria can be performed by a cell culture method known in the art, and may be appropriately selected depending on the type of target cells with which the complex has been contacted.

The culture conditions in this step, such as the culture temperature, CO₂ concentration, culture period, and medium exchange frequency, are not limited. For example, cells may be statically cultured at 37°C and 5% CO₂ for 30 minutes or more or 2 hours or more, such as 3 to 96 hours, 6 to 48 hours, or 9 to 24 hours.

The medium to be used for culture can be appropriately selected from known media. For example, in the case of animal cells, any liquid medium for culturing animal cells can be used as the basal medium, and other components (serum, serum replacement reagents, growth factors, etc.) can be appropriately added as necessary to prepare the medium.

### 6-3. Effects

In cells to which the carrier peptide / nucleic acid complex has been introduced by the nucleic acid introduction method of the present invention, the complex is transferred to mitochondria, and the gene encoded by the nucleic acid is expressed in the mitochondria. The transfer route of the carrier peptide / nucleic acid complex is not limited, but the complex can be transferred to mitochondria via endosomes/lysosomes, for example. In the nucleic acid introduction method of the present invention, the carrier peptide preferably comprises a mitochondrial-targeting sequence derived from the biological species from which the cells are derived. For example, for human cells, it is preferable to use a mitochondrial-targeting sequence derived from the human mitoNEET protein in order to increase the efficiency of introducing to mitochondria.

In the nucleic acid introduction method of the present invention, there is no upper limit for the size of a nucleic acid to be delivered, and even large nucleic acid molecules such as mtDNA can be introduced to mitochondria.

### Examples

### <Example 1: Preparation of carrier peptide / nucleic acid complex>

### (Purpose)

A carrier peptide is prepared by fusing a mitochondrial-targeting sequence with a polycationic sequence. Further, a carrier peptide / nucleic acid complex comprising the carrier peptide and a plasmid DNA is prepared.

### (Methods and results)

### (1) Preparation of carrier peptide

As a mitochondrial-targeting sequence contained in the carrier peptide, the amino acid sequence (MSLTSSSSVRVEWIAAVTIAAGTAAIGYLAYK; SEQ ID NO: 2) consisting of amino acids at positions 1 to 32 on the N-terminal side of a human mitoNEET protein (108 amino acids long) was used. This mitochondrial-targeting sequence is known as an anchor region that can penetrate the mitochondrial outer membrane, and comprises a transmembrane region consisting of the amino acid sequence (IAAVTIAAGTAAIGYLAY) shown in SEQ ID NO: 1. As the polycationic sequence contained in the carrier peptide, an RH repeating sequence (RHRHRHRHRHRHRHRHRH; SEQ ID NO: 3) or a KH repeating sequence (KHKHKHKHKHKHKHKHKH; SEQ ID NO: 4) was used.

In the following Examples, the carrier peptide (MSLTSSSSVRVEWIAAVTIAAGTAAIGYLAYKRHRHRHRHRHRHRHRHRH; SEQ ID NO: 5) prepared by fusing the mitochondrial-targeting sequence with the RH repeating sequence is referred to as "mitoNEET-(RH)₉". Also, the carrier peptide (MSLTSSSSVRVEWIAAVTIAAGTAAIGYLAYKKHKHKHKHKHKHKHKHKH; SEQ ID NO: 6) prepared by fusing the mitochondrial-targeting sequence with the KH repeating sequence is referred to as "mitoNEET-(KH)₉."

### (2) Preparation of carrier peptide / nucleic acid complex

A peptide solution (1 mg/mL) was prepared by dissolving mitoNEET-(RH)₉ or mitoNEET-(KH)₉ in ultrapure water. The peptide solution was mixed with a plasmid DNA solution at an N/P ratio of 2, 2.5, 3, or 4, the mixture was shaken at 1,200 rpm at room temperature for 30 minutes, and then the pH was adjusted with 10 mM HEPES buffer (pH 7.3) (final concentration: 2 mM). In this example, a 6.3 kbp plasmid DNA comprising a GFP gene was used as the plasmid DNA.

In the following Examples, a carrier peptide / nucleic acid complex comprising mitoNEET-(RH)₉ as a carrier peptide is referred to as a "mitoNEET-(RH)₉/nucleic acid complex." Further, a carrier peptide / nucleic acid complex comprising mitoNEET-(KH)₉ as a carrier peptide is referred to as a "mitoNEET-(KH)₉/nucleic acid complex."

### (3) Measurement of particle size and zeta potential

The particle size of the carrier peptide / nucleic acid complex was measured by dynamic light scattering (DLS). DLS measurement was performed using Zetasizer Nano (Malvern Instruments, UK) under conditions of 25°C and a scattering angle of 173°. The zeta potential of the carrier peptide / nucleic acid complex in 2 mM HEPES buffer was measured at 25°C by laser Doppler electrophoresis using Zetasizer Nano. The zeta potential was calculated based on the Smoluchowski equation.

The results are shown in Table 1 below. The mitoNEET-(RH)₉/nucleic acid complexes and the mitoNEET-(KH)₉/nucleic acid complexes formed at all N/P ratios exhibited cumulative diameters of 64 to 88 nm (polydispersity index PDI < 0.25). These results demonstrated efficient complex formation.

**[Table 1]**

| Table 1: Results of analyzing carrier peptide / nucleic acid complex | | | | |
|---|---|---|---|---|
| | N/P ratio | Size | PDI | Zeta potential |
| mitoNEET-(RH)₉ /nucleic acid complex | 2 | 78 ± 2.4 nm | 0.22 ± 0.01 | + 21.4 ± 2.9 mV |
| | 2.5 | 76 ± 3.0 nm | 0.24 ± 0.02 | + 21.4 ± 2.9 mV |
| | 3 | 83 ± 2.9 nm | 0.24 ± 0.01 | + 19.7 ± 2.1 mV |
| | 4 | 88 ± 6.9 nm | 0.22 ± 0.02 | + 20.1 ± 4.2 mV |
| mitoNEET-(KH)₉ /nucleic acid complex | 2 | 67 ± 1.6 nm | 0.16 ± 0.02 | + 29.0 ± 2.6 mV |
| | 2.5 | 77 ± 9.2 nm | 0.18 ± 0.01 | + 24.7 ± 1.0 mV |
| | 3 | 65 ± 1.0 nm | 0.17 ± 0.01 | + 28.3 ± 1.2 mV |
| | 4 | 64 ± 2.2 nm | 0.20 ± 0.01 | + 28.5 ± 0.7 mV |

| | | | | |
|---|---|---|---|---|
| * Values indicate mean ± SEM (n = 4). | | | | |

### <Example 2: Transfer of carrier peptide / nucleic acid complex to mitochondria>

### (Purpose)

The carrier peptide / nucleic acid complexes are introduced to HeLa cells to investigate that the carrier peptide / nucleic acid complexes can be transferred to mitochondria.

### (Methods and results)

### (1) Preparation of carrier peptide / nucleic acid complex

In the same manner as in Example 1, the mitoNEET-(RH)₉/nucleic acid complex and the mitoNEET-(KH)₉/nucleic acid complex were prepared under conditions of an N/P ratio of 2.

In addition, a carrier peptide comprising a cell-penetrating sequence instead of the mitochondrial-targeting sequence was prepared as a control. Specifically, the carrier peptide (SEQ ID NO: 8; hereinafter referred to as "Tat-(RH)₉") was prepared by fusing the cell-penetrating sequence (Tat) consisting of the amino acid sequence (RKKRRQRRR) shown in SEQ ID NO: 7 with the RH repeating sequence (RHRHRHRHRHRHRHRHRH; SEQ ID NO: 3), and then a carrier peptide / nucleic acid complex was prepared under the condition of N/P ratio = 2 in the same manner as in Example 1. Hereinafter, this carrier peptide / nucleic acid complex is referred to as "Tat-(RH)₉/nucleic acid complex".

In this example, Cy3-labeled plasmid DNA was used as the nucleic acid to observe the carrier peptide / nucleic acid complexes.

### (2) Localization analysis of carrier peptide / nucleic acid complex

HeLa cells were cultured in DMEM containing 10% FBS and 1% penicillin/streptomycin at 37°C. HeLa cells were seeded at 100,000 cells/dish on 35 mm glass-bottom dishes. After removing the medium, HeLa cells were transfected with the carrier peptide / nucleic acid complex comprising 10 µg of Cy3-labeled plasmid (600 µL). After 15 minutes of incubation, a fresh medium (1 mL) was added. In this and the following examples, transfection was performed by incubating the carrier peptide / nucleic acid complex with HeLa cells for a certain period of time, and no manipulation such as lipofection was performed.

After transfection, HeLa cells were incubated for 3 to 24 hours and then washed twice with D-PBS. The cells were stained with 100 nM MitoVIEW^{™} 405 (Thermo Fisher Scientific) and 100 nM LysoTracker Green (Thermo Fisher Scientific) for 30 minutes, followed by treatment with a 0.05% trypan blue solution to quench the fluorescent dye on the cell surface. The carrier peptide / nucleic acid complex (Cy3-labeled plasmid/561 nm), mitochondria (MitoVIEW^{™} 405/405 nm), and late endosomes/lysosomes (LysoTracker Green/561 nm) were observed 3, 6, and 24 hours after transfection using a spinning disk confocal super resolution microscope (CLSM, Olympus).

The colocalization ratio of the Cy3-labeled plasmid colocalized with mitochondria or late endosomes/lysosomes was calculated using the following formula for each of the 35 cells with ImageJ software. Colocalization ratiomito = (the number of Cy3 pixels colocalized with mitochondria)/(total number of Cy3 pixels) Colocalization ratiolyso = (the number of Cy3 pixels colocalized with late endosomes/lysosomes)/(total number of Cy3 pixels)

The colocalization ratioₘᵢₜₒ and the colocalization ratio_{lyso} of the mitoNEET-(RH)₉ /nucleic acid complexes at 3, 6, and 24 hours after transfection are shown in Figure 1A. At 3 hours after transfection, the majority of the mitoNEET-(RH)₉/nucleic acid complexes remained trapped in late endosomes/lysosomes, whereas at 6 hours after transfection, approximately half of the mitoNEET-(RH)₉/nucleic acid complexes escaped from the endosomal compartment and reached mitochondria. The colocalization ratios at 24 hours after transfection were equivalent to those at 6 hours after transfection. Thus, escape from endosomes and transfer to mitochondria were demonstrated to occur within 6 hours after transfection.

Next, for the colocalization ratioₘᵢₜₒ and the colocalization ratio_{lyso} 6 hours after transfection, the results of comparing the mitoNEET-(RH)₉/nucleic acid complex, the mitoNEET-(KH)₉/nucleic acid complex, and the TAT-(RH)₉/nucleic acid complex are shown in Figure 1B. The Tat-(RH)₉/nucleic acid complex, whose carrier peptide comprises the cell-penetrating sequence instead of the mitochondrial-targeting sequence, exhibited endosomal escape ability equivalent to that of the mitoNEET-(RH)₉/nucleic acid complex, but its accumulation level in mitochondria was lower than that of the mitoNEET-(RH)₉/nucleic acid complex. On the other hand, the mitoNEET-(KH)₉/nucleic acid complex exhibited a significantly higher accumulation level in mitochondria than that of the Tat-(RH)₉/nucleic acid complex, although the level was lower than that of the mitoNEET-(RH)₉/nucleic acid complex.

These results revealed that the mitoNEET-(RH)₉/nucleic acid complex and the mitoNEET-(KH)₉/nucleic acid complex exhibit efficient transfer to mitochondria.

### (3) Time-lapse imaging of carrier peptide / nucleic acid complexes

HeLa cells were seeded at 100,000 cells/dish on 35-mm glass-bottom dishes. The cells were stained with 100 nM MitoBright deep red (Dojindo, Japan) for 30 minutes, washed twice with D-PBS, and then transfected with the mitoNEET-(RH)₉/nucleic acid complex comprising 10 µg of Cy3-labeled plasmid (600 µL) in the same manner as in (2) above. After 15 minutes of incubation, 1 mL of a fresh medium was added. 2.5 hours after transfection, the cells were treated with a 0.05% trypan blue solution to quench the fluorescent dye on the cell surface, and then images of the mitoNEET-(RH)₉/nucleic acid complex (Cy3-labeled plasmid/561 nm) and mitochondria (MitoBright deep red/640 nm) were taken for 5 minutes every 15 seconds using a spinning disk confocal super resolution microscope.

Time-lapse images capturing the transfer process of the mitoNEET-(RH)₉/nucleic acid complex to mitochondria are shown in Figure 2. Surprisingly, the size and morphology of green dots representing the Cy3-labeled plasmid were maintained at 100 nm to 200 nm throughout the incorporation process. Although the mitochondrial outer membrane is thought to lack a mechanism for incorporation of submicron-sized macromolecules, this result revealed that the mitoNEET-(RH)₉/nucleic acid complex could be transferred to mitochondria without dissociation.

### <Example 3: Protein expression in mitochondria based on carrier peptide / nucleic acid complex>

### (Purpose)

A carrier peptide / nucleic acid complex comprising a plasmid DNA encoding GFP is introduced to HeLa cells. It is investigated that a protein can be expressed from the nucleic acid in the carrier peptide / nucleic acid complex that has been transferred to mitochondria.

### (Methods and results)

### (1) Construction of pmtGFP plasmid and pnuclearGFP plasmid

To evaluate the expression of exogenous genes in mitochondria, two types of plasmids, "pmtGFP plasmid" and "pnuclearGFP plasmid", in which a GFP gene was placed under the control of a mitochondria-specific cyclooxygenase 2 (cox2) promoter, were constructed.

In the pmtGFP plasmid, a TGG codon (nucleotide at position 172 to nucleotide at position 174 in the GFP gene) corresponding to amino acid at position 58 in GFP has been substituted with a TGA codon. The TGA codon encodes tryptophan in mitochondrial codons, but is a stop codon in nuclear codons. Therefore, the GFP gene on the pmtGFP plasmid can emit GFP fluorescence only when translated in mitochondria.

In the pnuclearGFP plasmid used as a control, a CGC codon (nucleotide at position 220 to nucleotide at position 222 in the GFP gene) corresponding to amino acid at position 74 in GFP has been substituted with an AGA codon. The AGA codon is a stop codon in mitochondrial codons, but encodes arginine in nuclear codons. Therefore, the GFP gene on the pnuclearGFP plasmid can emit GFP fluorescence only when translated in the cytoplasm, and cannot emit GFP fluorescence when translated in mitochondria.

The above two types of plasmids were constructed by site-directed mutagenesis to the pDONR-cox2: gfp plasmid (5.1 kbp) described in previous literature (Chuah, J.A. et al., Scientific Reports, 2015, 5:7751; Chuah, J.A. et al., Biomacromolecules, 2016, 17:3547-3557).

### (2) Analysis of GFP expression in mitochondria

A carrier peptide / nucleic acid complex was prepared under the condition of an N/P ratio of 2 in the same manner as in Example 1 by using the pmtGFP plasmid and the pnuclearGFP plasmid as the nucleic acids, and mitoNEET-(RH)₉ as the carrier peptide in this example. Hereinafter, the carrier peptide / nucleic acid complex comprising the pmtGFP plasmid as the nucleic acid is referred to as the "mitoNEET-(RH)₉/pmtGFP complex." In addition, the carrier peptide / nucleic acid complex comprising the pnuclearGFP plasmid as the nucleic acid is referred to as the "mitoNEET-(RH)₉/pnuclearGFP complex."

HeLa cells were seeded at 100,000 cells/dish on 35 mm glass-bottom dishes. After removing the medium, HeLa cells were transfected with the carrier peptide / nucleic acid complex containing 10 µg of plasmid DNA. After 15 minutes of incubation, 1 mL of a fresh medium was added. After 24 hours, the medium was removed and fresh medium was added to the dishes. After another 24 hours of incubation, the cells were washed twice with D-PBS and stained with 50 nM MitoTracker red and 100 nM LysoTracker deep red for 30 minutes. After treating the cells with a 0.05% trypan blue solution to quench the fluorescent dye on the cell surface, GFP (488 nm), mitochondria (MitoTracker red/561 nm), and late endosomes/lysosomes (LysoTracker Deep red/640 nm) were observed using a spinning disk confocal super resolution microscope.

The results are shown in Figure 3A. The mitoNEET-(RH)₉/pmtGFP complex exhibited bright green fluorescence in mitochondria. In contrast, the mitoNEET-(RH)₉/pnuclearGFP complex did not exhibit green fluorescence in mitochondria. This result indicated that the mitoNEET-(RH)₉/pmtGFP complex was delivered to mitochondria and that GFP was efficiently expressed in mitochondria from the pmtGFP plasmid.

### (3) Flow cytometry analysis of GFP-expressing cells

The mitoNEET-(RH)₉/pmtGFP complex was prepared in the same manner as in (2) above. As a control, the carrier peptide / nucleic acid complex comprising the pmtGFP plasmid in (1) above and Tat-(RH)₉ described in Example 2(1) (hereinafter referred to as "Tat-(RH)₉/pmtGFP complex") was prepared in the same manner.

HeLa cells were seeded at 100,000 cells/well on 6-well plates. After 6, 24, 48, 72, and 96 hours of culture, the medium was removed, and the HeLa cells were transfected with the carrier peptide / nucleic acid complex containing 10 µg of plasmid DNA. After 15 minutes of incubation, 1 mL of fresh culture medium was added and incubated for additional 6 hours. The cells were washed twice with D-PBS and then collected by trypsinization. GFP intensity and the percentage of GFP-positive cells were determined using BD LSR (Franklin Lakes, NJ) using 488 nm laser excitation with a 530/28 BP filter.

The results of flow cytometry after 48 hours of culture are shown in Figure 3B. Strong expression of GFP was detected for the mitoNEET-(RH)₉/pmtGFP complex, whereas almost no expression of GFP was detected for the Tat-(RH)₉/pmtGFP complex. In flow cytometry after 6 to 96 hours of culture, the GFP intensity reached its maximum after 48 hours.

### <Example 4: Restoration of mitochondrial function by introduction of mitochondrial DNA>

### (Purpose)

A carrier peptide / nucleic acid complex comprising mitochondrial DNA (hereinafter abbreviated as "mtDNA") is introduced to Hela cells depleted of mtDNA (hereinafter referred to as "Hela-ρ⁰ cells").

### (Methods and results)

### (1) Preparation of Hela-ρ⁰ cells

Hela-ρ⁰ cells were prepared by ethidium bromide treatment according to the method described in the literature (King, M. P. & Attardi, G. in Methods in Enzymology, Vol. 264: 304-313 (Academic Press, 1996)). Hela-ρ⁰ cells were cultured at 37°C in DMEM comprising 10% FBS, 1% penicillin/streptomycin, 2 mM L-glutamine, 1 mM sodium pyruvate, and 50 µg/mL uridine.

### (2) Preparation of mtDNA

mtDNA used herein was prepared by inserting an OriC cassette for *in vitro* amplification into mtDNA extracted from a normal cell line (HEK293 cells) based on the method described in the literature (Su'etsugu, M. et al., Nucleic Acids Res, 2017, 45:11525-11534; Hasebe, T et al., Life (Basel), 2018, 8:43) (Figure 4). Specifically, wild-type mtDNA was isolated from HEK293 cells using a QIAprep Spin Miniprep Kit (QIAGEN, Germany), mtDNA was digested with Xho I, and then RA-seamless overlapping assembly and RCR amplification using the OriC cassette were performed according to the above literature.

### (3) Preparation of mitoNEET-(RH)₉/mtDNA complex

In the same manner as in Example 1, a carrier peptide / nucleic acid complex comprising mitoNEET-(RH)₉ as the carrier peptide and mtDNA as the nucleic acid (hereinafter referred to as "mitoNEET-(RH)₉/mtDNA complex") was prepared at an N/P ratio of 2.

The particle size of the mitoNEET-(RH)₉/mtDNA complex was measured by dynamic light scattering (DLS), and the zeta potential was measured in the same manner as in Example 1. As a result, the mitoNEET-(RH)₉/mtDNA complex exhibited a slightly larger cumulative diameter and a similar zeta potential compared to the pmtGFP/mtDNA complex (size 155 ± 6.3 nm, PDI = 0.22 ± 0.01, zeta potential 16.2 ± 0.3 mV). The difference in complex size is considered to be due to the difference in nucleic acid size (pmtGFP 6.3 kbp vs. mtDNA 16.6 kbp).

### (4) Gene expression from mtDNA

The mitoNEET-(RH)₉/mtDNA complex was introduced to Hela-ρ⁰ cells, and the expression levels of ND1 mRNA and ND5 mRNA from mtDNA were measured.

Specifically, Hela-ρ⁰ cells were seeded at 5,000 cells/well on 96-well plates. After 24 hours of culture, the medium was removed, and the mitoNEET-(RH)₉/mtDNA complex (30 µL) containing 500 ng of mtDNA was added. After 15 minutes, 70 µL of fresh medium was added to each well. Twenty four (24) hours after transfection, the medium was removed and fresh medium was added to the dishes. After another 24 hours of incubation, the cells were lysed and total RNA was extracted using an RNeasy mini kit (Qiagen, Germany), followed by reverse transcription using a ReverTra Ace kit (TAKARA). Quantitative real-time PCR was performed using primer pairs corresponding to each gene (ND1 mRNA: SEQ ID NO: 9 and 10, ND5 mRNA: SEQ ID NO: 11 and 12, β-actin mRNA: SEQ ID NO: 13 and 14) and a StepOnePlus Real-Time PCR System.

The expression levels of ND1 mRNA and ND5 mRNA were quantified by qRT-PCR and the results are shown in Figure 5A. The expression levels of ND1 mRNA and ND5 mRNA in Hela-ρ⁰ cells transfected with the mitoNEET-(RH)₉/mtDNA complex were 3,100 times and 28 times higher, respectively, than those in untreated Hela-ρ⁰ cells and were equivalent to those in wild-type HeLa cells. This result suggested that transfection with the mitoNEET-(RH)₉/mtDNA complex restored mitochondrial function in Hela-ρ⁰ cells.

### (5) Quantification of NADH level

To evaluate mitochondrial function in Hela-ρ⁰ cells transfected with the mitoNEET-(RH)₉/mtDNA complex, the level of the electron donor NADH was measured. Since Hela-ρ⁰ cells cannot produce energy in mitochondria, they require pyruvic acid and uridine in the medium for cell growth. Therefore, the effect of the mitoNEET-(RH)₉/mtDNA complex was investigated by measuring metabolic activity in a low-supplement medium with low concentrations of pyruvate and uridine.

Specifically, Hela-ρ⁰ cells were seeded at 5,000 cells/well on 96-well plates. After 24 hours of culture, the medium was removed, and the mitoNEET-(RH)₉/mtDNA complex (30 µL) containing 500 µg of mtDNA was added. After 15 minutes, 70 µL of complete culture medium was added to each well, and after 24 hours, the medium was replaced with 400 µL of low-supplement medium (2 mM L-glutamine, 4 µg/mL sodium pyruvate, and 50 µg/mL uridine). Transfection was repeated every 2 days for a total of three times by the same procedure as above. The medium was replaced with fresh low-supplement medium every day. The NADH level was evaluated every other day by measuring the absorbance at 450 nm with a SpectraMax iD3 microplate reader using a CCK-8 assay kit.

The results of measuring NADH levels are shown in Figure 5B. The relative NADH value in Hela-ρ⁰ cells transfected with the mitoNEET-(RH)₉/mtDNA complex was significantly higher than that in untreated Hela-ρ⁰ cells from 7 to 15 days after transfection. On the other hand, the NADH level was not improved in Hela-ρ⁰ cells transfected with the mitoNEET-(RH)₉/pmtGFP complex.

The above results demonstrated that the introduction of the mitoNEET-(RH)₉/mtDNA complex can improve mitochondrial energy production.

### (6) Evaluation of OCR and ECAR

To further evaluate mitochondrial function in Hela-ρ⁰ cells transfected with the mitoNEET-(RH)₉/mtDNA complex, the oxygen consumption rate (OCR), which correlates with mitochondrial respiration, and the extracellular acidification rate (ECAR), which correlates with the glycolytic state, were evaluated using a flux analyzer.

Specifically, Hela-ρ⁰ cells were seeded at 50,000 cells/well on 24-well plates. After 24 hours of incubation, the medium was removed, and Hela-ρ⁰ cells (300 µL) containing 4 µg of mtDNA were added. After 15 minutes, 300 µL of complete culture medium was added, and 24 hours later, the medium was replaced with 400 µL of low-supplement medium (2mM L-glutamine, 4 µg/mL sodium pyruvate, and 50 µg/mL uridine). Transfection was repeated every two days for a total of three times by the same procedure as above. Six (6) days after the initial transfection, cells were collected by trypsinization and re-seeded at 20,000 cells/well on XF96 cell culture microplates (Agilent, CA) followed by an additional 24 hours of incubation in low-supplement medium. The medium was replaced with XF-DMEM Seahorse Agilent comprising 1% FBS and 4.5 g/L glucose and the cells were incubated at 37°C for 1 hour. A sensor cartridge in a XFe96 extracellular flux assay kit was set up, and then the OCR and ECAR were measured using a Seahorse XFe96 analyzer (Agilent).

The oxygen consumption rate (OCR) and extracellular acidification rate (ECAR) were measured, and the results are shown in Figure 6. The OCR and ECAR in Hela-ρ⁰ cells transfected with the mitoNEET-(RH)₉/mtDNA complex were significantly higher than those in untreated Hela-ρ⁰ cells. These results demonstrated that introduction of the mitoNEET-(RH)₉/mtDNA complex can improve mitochondrial energy production.

### (7) Mitochondrial morphology

The mitochondrial morphology in Hela-ρ⁰ cells transfected with the mitoNEET-(RH)₉/mtDNA complex was evaluated based on the aspect ratio and branching value.

Specifically, Hela-ρ⁰ cells were seeded at 20,000 cells/well on glass-bottom 24-well plates. After 24 hours of incubation, the medium was removed and the mitoNEET-(RH)₉/mtDNA complex (120 µL) containing 2 µg of mtDNA was added. After 15 minutes, 280 µL of complete culture medium was added, and after 24 hours the medium was replaced with 400 µL of the above low-supplement medium. Transfection was repeated every three days for a total of three times by the same procedure as above. Seven (7) days after the initial transfection, the cells were stained with MitoTracker red (50 nM) for 30 minutes. The cells were observed using a spinning disk confocal super resolution microscope. Mitochondrial morphology was evaluated based on the aspect ratio and branching value. The Aspect Ratio (AR) was calculated as the ratio of the major axis to the minor axis. The branching value (form factor, FF) was calculated as (length of mitochondrial perimeter)²/(4π × (area of mitochondria)).

The results of measuring the aspect ratio and the branching value are shown in Figure 7. The aspect ratio and the branching value in Hela-ρ⁰ cells transfected with the mitoNEET-(RH)₉/mtDNA complex were significantly higher than those in untreated Hela-ρ⁰ cells. These results also demonstrated the restoration of mitochondrial function in Hela-ρ⁰ cells through the introduction of the mitoNEET-(RH)₉/mtDNA complex.

### <Example 5: Performance comparison of mitochondrial-targeting sequence>

### (Purpose)

As a mitochondrial-targeting sequence which is different from mitoNEET, a carrier peptide was prepared by fusing 12 amino acid residues derived from yeast cytochrome c oxidase subunit IV (Cytocox) with a polycationic sequence, and then compared with mitoNEET-(RH)₉ for performance.

### (Methods and results)

### (1) Preparation of Cytocox-(KH)₉/pmtGFP complex

As the mitochondrial-targeting sequence, a carrier peptide (MLSLRQSIRFFKKHKHKHKHKHKHKHKHKH; SEQ ID NO: 16; hereafter referred to as "Cytocox-(KH)₉") was prepared by fusing 12 amino acid residues (MLSLRQSIRFFK; SEQ ID NO: 15) derived from yeast cytochrome c oxidase subunit IV (Cytocox) with a KH repeating sequence. A carrier peptide / nucleic acid complex comprising this carrier peptide and a pmtGFP plasmid (hereinafter referred to as "Cytocox-(KH)₉/pmtGFP complex") was prepared in the same manner as in Example 1 under the condition of an N/P ratio of 2.

### (2) Measurement of particle size and zeta potential

The particle size of the carrier peptide / nucleic acid complex was measured by dynamic light scattering (DLS) and the zeta potential was measured in the same manner as in Example 1.

The results are shown in Table 2 below. The Cytocox-(KH)₉/pmtGFP complex exhibited a cumulative diameter of approximately 70 nm to 80 nm, similar to that of the mitoNEET-(RH)₉/pmtGFP complex. The mitoNEET-(RH)₉/pmtGFP complex exhibited a higher zeta potential than that of the Cytocox-(KH)₉/pmtGFP complex.

**[Table 2]**

| Table 2: Results of measuring particle size and zeta potential | | | |
|---|---|---|---|
| | Size | PDI | Zeta potential |
| mitoNEET-(RH)₉/pmtGFP complex | 70 ± 0.8 nm | 0.17 | + 29 ± 0.6 mV |
| Cytocox-(KH)₉/pmtGFP complex | 74 ± 1.0 nm | 0.23 | + 11 ± 1.8 mV |

### (3) Analysis of mitochondrial GFP expression

HeLa cells were seeded at 100,000 cells/well on 24 mm glass-bottom dishes. After 24 hours of culture, the medium was removed, and the carrier peptide / nucleic acid complex (120 µL) containing 2 µg of plasmid DNA was added to each well. After 15 minutes of incubation, 280 µL of complete medium (1 mL) was added to each well. After 24 hours, the medium was removed and fresh medium was added to the dishes. Seventy two (72) hours after transfection, MitoTracker red CMXros (final concentration: 50 nM) was added to each well, incubation was performed for 15 minutes, and then the resultants were observed using a spinning disk confocal super resolution microscope.

The results are shown in Figure 8. It was revealed that compared with cells transfected with the Cytocox-(KH)₉/pmtGFP complex, GFP was significantly more strongly expressed in HeLa cells transfected with the mitoNEET-(RH)₉/pmtGFP complex. This result demonstrated that the mitoNEET-derived mitochondrial-targeting sequence has significantly superior performance of introducing to mitochondria compared with the Cytocox-derived mitochondrial-targeting sequence.

### <Example 6: Protein expression in mitochondria based on carrier peptide/mRNA complex>

### (Purpose)

A carrier peptide/mRNA complex comprising messenger RNA (mRNA) encoding GFP is introduced to HeLa cells. It is investigated that GFP can be expressed from the mRNA in the carrier peptide/mRNA complex that has been transferred to mitochondria.

### (Methods and results)

### (1) Preparation of mRNA

The mRNA encoded by the GFP gene in the pmtGFP plasmid used in Example 3 was prepared by *in vitro* transcription. In this mRNA, a TGG codon (nucleotide at position 172 to nucleotide at position 174 in the GFP gene) corresponding to amino acid at position 58 in GFP had been replaced with a TGA codon. The TGA codon encodes tryptophan in mitochondrial codons, but is a stop codon in nuclear codons. Therefore, GFP encoded by the mRNA introduced to cells can emit GFP fluorescence only when translated in mitochondria.

### (2) Analysis of GFP expression in mitochondria

A carrier peptide/mRNA complex was prepared under the condition of an N/P ratio of 2 in the same manner as in Example 1. However, in this example, the mRNA prepared in (1) above was used as the nucleic acid, and mitoNEET-(RH)₉ was used as the carrier peptide. Hereinafter, the prepared carrier peptide/mRNA complex is referred to as the "mitoNEET-(RH)₉/mRNA complex."

HeLa cells were seeded at 100,000 cells/dish on 35 mm glass-bottom dishes. After removing the medium, HeLa cells were transfected with the mitoNEET-(RH)₉/mRNA complex containing 10 µg of the above mRNA. After 15 minutes of incubation, 1 mL of fresh medium was added. After 24 hours, the medium was removed and fresh medium was added to the dishes. After another 24 hours of incubation, the cells were washed twice with D-PBS and stained with 50 nM MitoTracker red for 30 minutes. GFP (488 nm) and mitochondria (MitoTracker red/561 nm) were observed using a spinning disk confocal super resolution microscope.

The results are shown in Figure 9. GFP expression was observed in the mitochondria of HeLa cells transfected with the mitoNEET-(RH)₉/mRNA complex. These results demonstrated that the mitoNEET-(RH)₉/mRNA complex was delivered to mitochondria and that GFP was translated from the mRNA in the mitochondria.

All publications, patents, and patent applications cited herein are hereby incorporated by reference in their entirety.

## Claims

1. A carrier peptide for introducing a nucleic acid to mitochondria, comprising a mitochondrial-targeting sequence and a polycationic sequence,
wherein said mitochondrial-targeting sequence consists of an N-terminal region comprising the transmembrane domain in a mitoNEET protein.

2. The carrier peptide of claim 1, wherein said mitochondrial-targeting sequence consists of the amino acid sequence MSLTSSSSVRVEWIAAVTIAAGTAAIGYLAYK (SEQ ID NO: 2).

3. The carrier peptide of claim 1, wherein said polycationic sequence comprises at least three cationic amino acid residues.

4. The carrier peptide of claim 3, wherein said cationic amino acid residues are lysine (K) residues, arginine (R) residues, histidine (H) residues, and/or derivatives of any of them.

5. The carrier peptide of claim 1, wherein said polycationic sequence comprises:
five or more consecutive lysine (K) residues, arginine (R) residues, histidine (H) residues, or derivatives of any of them;
seven or more consecutive lysine (K) residues, arginine (R) residues, histidine (H) residues, and/or derivatives of any of them; or
a sequence formed by repeating the amino acid sequence KH or RH three to twenty times.

6. A nucleic acid encoding the carrier peptide of any one of claims 1 to 5.

7. A carrier peptide / nucleic acid complex for introducing a nucleic acid to mitochondria, comprising the carrier peptide of any one of claims 1 to 5 and the nucleic acid.

8. The carrier peptide / nucleic acid complex of claim 7, wherein the number of amino groups and guanidino groups derived from said carrier peptide / the number of phosphate groups derived from said nucleic acid (N/P ratio) is 0.2 or more and 100 or less.

9. The carrier peptide / nucleic acid complex of claim 7, wherein said nucleic acid encodes a mitochondrial protein.

10. The carrier peptide / nucleic acid complex of claim 7, wherein said nucleic acid is DNA or RNA.

11. The carrier peptide / nucleic acid complex of claim 10, wherein said DNA is 1 kbp to 20 kbp.

12. The carrier peptide / nucleic acid complex of claim 10, wherein said DNA is a mitochondrial DNA.

13. A pharmaceutical composition for treating a mitochondrial disease, comprising the carrier peptide / nucleic acid complex of claim 7.

14. A method of producing a carrier peptide / nucleic acid complex, comprising:
a complex formation step of mixing the carrier peptide of any one of claims 1 to 5 with a nucleic acid to form a carrier peptide / nucleic acid complex comprising said carrier peptide and said nucleic acid.

15. A method of introducing a nucleic acid to mitochondria of an isolated target cell, comprising:
a contact step of contacting the carrier peptide / nucleic acid complex of claim 7 with an isolated target cell, and
a transfer step of culturing said target cell after said contact step to transfer said carrier peptide / nucleic acid complex to mitochondria.
